(19) Europäisches Patentamt / European Patent Office / Office européen des brevets

(11) **EP 2 585 615 B1**

(12) **EUROPEAN PATENT SPECIFICATION**

(45) Date of publication and mention of the grant of the patent:
**30.03.2016 Bulletin 2016/13**

(21) Application number: **11736024.8**

(22) Date of filing: **22.06.2011**

(51) Int Cl.:
*C12Q 1/68* (2006.01)

(86) International application number:
**PCT/EP2011/060524**

(87) International publication number:
**WO 2011/161199 (29.12.2011 Gazette 2011/52)**

(54) **HIGHLY SENSITIVE METHOD FOR DETECTION OF A TARGET NUCLEIC ACID IN A SAMPLE**

HOCHEMPFINDLICHES VEFAHREN FÜR DEN NACHWEIS EINER ZIELNUKLEINSÄURE IN EINER PROBE

MÉTHODE DE DÉTECTION DE FORTE SENSIBILITÉ D'UN ACIDE NUCLÉIQUE CIBLE DANS UN ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.06.2010 IT MI20101132**

(43) Date of publication of application:
**01.05.2013 Bulletin 2013/18**

(73) Proprietor: **Euroclone Diagnostica S.r.l.**
**20142 Milano (IT)**

(72) Inventors:
• **SPINELLI, Luigi**
**I-22060 Mariano Comense (IT)**

• **BOSIO, Paolo**
**I-12051 Alba (IT)**
• **CANFORA, Paola**
**I-22069 Rovellasca (IT)**
• **PIONA, Riccardo**
**20162 Milano (IT)**
• **PIZZIGONI, Alessandro**
**I-20122 Milan (IT)**

(74) Representative: **Croce, Valeria**
**Jacobacci & Partners S.p.A.**
**Via Senato, 8**
**20121 Milano (IT)**

(56) References cited:
EP-A2- 1 942 196       WO-A1-03/054233
WO-A2-01/96612       WO-A2-2006/044994
WO-A2-2007/149903       JP-A- 2005 211 032

EP 2 585 615 B1

SEQUENCE LISTING

<110> EUROCLONE S.P.A.

<120> HIGH SENSITIVITY METHOD FOR DETECTING A TARGET NUCLEIC ACID SEQUENCE IN A SAMPLE

<130> 11140PTWO

<150> MI2010A001132
<151> 2010-06-22

<160> 69

<170> Patent In version 3.5

<210> 1
<211> 26
<212> DNA
<213> Clamydia phage 1

<400> 1
tggcgatatt tgggcatccg agtaac 26

<210> 2
<211> 28
<212> DNA
<213> Clamydia phage 1

<400> 2
tacgctcaaa tccagcgggt attaaccg 28

<210> 3
<211> 17
<212> DNA
<213> Clamydia phage 1

<400> 3
accgaaaggt cctaaga 17

<210> 4
<211> 19
<212> DNA
<213> Clamydia phage 1

<400> 4
acagagcgaa ccaagaatg 19

<210> 5
<211> 19
<212> DNA
<213> Clamydia phage 1

<400> 5
tagcctggat ctccttggt 19

<210> 6
<211> 21
<212> DNA
<213> Clamydia phage 1

<400> 6
aggaaactgc ttcctcagca g          21

<210> 7
<211> 20
<212> DNA
<213> Hepatitis C virus

<400> 7
cctcccggga gagccatagt 20

<210> 8
<211> 23
<212> DNA
<213> Hepatitis C virus

<400> 8
atgrtgcacg gtctacgaga cct 23

<210> 9
<211> 16
<212> DNA
<213> Hepatitis C virus

<400> 9
tgtactcacc ggttcc 16

<210> 10
<211> 24
<212> DNA
<213> Hepatitis B virus

<400> 10
tcactcacca acctsytgtc ctcc 24

<210> 11
<211> 22
<212> DNA
<213> Hepatitis B virus

<400> 11
aggacamacg ggcaacatac ct 22

<210> 12
<211> 18
<212> DNA
<213> Hepatitis B virus

<400> 12
tatcgytgga tgtgtctg 18

<210> 13
<211> 21
<212> DNA
<213> Toxoplasma gondii

<400> 13
tgtgcttgga gccacagaag g        21

<210> 14
<211> 22
<212> DNA
<213> Toxoplasma gondii

<400> 14
tcgtctrgat cgcattccgg tg 22

<210> 15
<211> 22
<212> DNA
<213> Toxoplasma gondii

<400> 15
agagatatca ggactgtaga tg 22

<210> 16
<211> 23
<212> DNA
<213> Herpes simplex virus 1

<400> 16
tgttctcgtt cctyacwgcc tcc        23

<210> 17
<211> 22
<212> DNA
<213> Herpes simplex virus 1

<400> 17
acgtaacgca cgctmgggtg tg 22

<210> 18
<211> 17
<212> DNA
<213> Herpes simplex virus 1

<400> 18
tccacacctt atcgttt 17

<210> 19
<211> 28
<212> DNA
<213> Herpes simplex virus 2

<400> 19
gatatcctct ttatcatcag caccacca 28

<210> 20
<211> 19

<212> DNA
<213> Herpes simplex virus 2

<400> 20
gtaacgcacg ctmgggtgc          19

<210> 21
<211> 16
<212> DNA
<213> Herpes simplex virus 2

<400> 21
accagacaaa cgaacg          16

<210> 22
<211> 18
<212> DNA
<213> Varicella zoster

<400> 22
gaagttcccc ccgttcgc          18

<210> 23
<211> 29
<212> DNA
<213> Varicella zoster

<400> 23
cctggacttg aagatgaact taatgaagc          29

<210> 24
<211> 21
<212> DNA
<213> Varicella zoster

<400> 24
acaactgcag tatatatcgt c 21

<210> 25
<211> 21
<212> DNA
<213> Epstein-Barr virus

<400> 25
aaacctcagg acctacgctg c 21

<210> 26
<211> 21
<212> DNA
<213> Epstein-Barr virus

<400> 26
cacagacacc gtcctcacca c 21

<210> 27
<211> 18
<212> DNA
<213> Epstein-Barr virus

<400> 27
tagaggtttt gctaggga        18

<210> 28
<211> 16
<212> DNA
<213> Citomegalovirus

<400> 28
agcgccgcat tgagga 16

<210> 29
<211> 21
<212> DNA
<213> Citomegalovirus

<400> 29
cagactctca gaggatcggc c 21

<210> 30
<211> 18
<212> DNA
<213> Citomegalovirus

<400> 30
atctgcatga aggtcttt 18

<210> 31
<211> 27
<212> DNA
<213> Herpes simplex virus 6

<400> 31
tgttaggrta taccgatgtg cgtgatc 27

<210> 32
<211> 22
<212> DNA
<213> Herpes simplex virus 6

<400> 32
cacgtaagct tgcacaatgc ca 22

<210> 33
<211> 18
<212> DNA
<213> Herpes simplex virus 6

<400> 33
ttccraatct attgcctc 18

<210> 34
<211> 22
<212> DNA
<213> herpes simplex virus 7

<400> 34
cggcgtttta ctcggaactc ct 22

<210> 35
<211> 25
<212> DNA
<213> herpes simplex virus 7

<400> 35
tccccataac aaatgtgcca taaga 25

<210> 36
<211> 19
<212> DNA
<213> herpes simplex virus 7

<400> 36
tacgattcag attttgtcc 19

<210> 37
<211> 22
<212> DNA
<213> Herpes simplex virus 8

<400> 37
agccgaaagg attccaccat tg 22

<210> 38
<211> 26
<212> DNA
<213> Herpes simplex virus 8

<400> 38
ggtggtatat agatcaagtt ccgcca 26

<210> 39
<211> 20
<212> DNA
<213> Herpes simplex virus 8

<400> 39
acatctactc caaaatatcg 20

<210> 40
<211> 19
<212> DNA
<213> Enterovirus

<400> 40
gcccctgaat gcggctaat 19

<210> 41
<211> 19
<212> DNA
<213> Enterovirus

<400> 41
caccggatgg ccaatccaa 19

<210> 42
<211> 17

<212> DNA
<213> Enterovirus

<400> 42
acccaaagta gtcggtt        17

<210> 43
<211> 27
<212> DNA
<213> BK virus

<400> 43
aatgcagtag caatctatcc aaaccaa 27

<210> 44
<211> 26
<212> DNA
<213> BK virus

<400> 44
gcaaggawtg gcctatttgt tccaaa 26

<210> 45
<211> 19
<212> DNA
<213> BK virus

<400> 45
atgccttaat ctaagctga        19

<210> 46
<211> 23
<212> DNA
<213> JC virus

<400> 46
agagtgytgg gatcctgtgt ttt        23

<210> 47
<211> 26
<212> DNA
<213> JC virus

<400> 47
atgaraagtg ggatgaagac ctgttt 26

<210> 48
<211> 18
<212> DNA
<213> JC virus

<400> 48
tggcaaacat ttcttcat 18

<210> 49
<211> 26
<212> DNA
<213> Chlamydia trachomatis

<400> 49
tggcgatatt tgggcatccg agtaac 26

<210> 50
<211> 28
<212> DNA
<213> Chlamydia trachomatis

<400> 50
tacgctcaaa tccagcgggt attaaccg 28

<210> 51
<211> 17
<212> DNA
<213> Chlamydia trachomatis

<400> 51
accgaaaggt cctaaga 17

<210> 52
<211> 30
<212> DNA
<213> Chlamydia trachomatis

<400> 52
aggatcactt ctcctcaaca actttttcat 30

<210> 53
<211> 27
<212> DNA
<213> Chlamydia trachomatis

<400> 53
tttgtagagt ttggttgggg aggttta 27

<210> 54
<211> 19
<212> DNA
<213> Chlamydia trachomatis

<400> 54
ttctgcttac aatgctctt 19

<210> 55
<211> 25
<212> DNA
<213> Clostridium difficile

<400> 55
caatgggctg atattaatgc agaat 25

<210> 56
<211> 30
<212> DNA
<213> Clostridium difficile

<400> 56
tcatctattg targtactgt aggtttattg 30

<210> 57
<211> 21
<212> DNA
<213> Clostridium difficile

<400> 57
acagctacta gaggaagaga t 21

<210> 58
<211> 26
<212> DNA
<213> Clostridium difficile

<400> 58
tgaggacata tcagagactg atgagg 26

<210> 59
<211> 30
<212> DNA
<213> Clostridium difficile

<400> 59
tagcatattc agagaatatt gyyttttcag 30

<210> 60
<211> 23
<212> DNA
<213> Clostridium difficile

<400> 60
ctggagaatc tatatttgta gaa 23

<210> 61
<211> 23
<212> DNA
<213> Mycoplasma genitalium

<400> 61
gagaaatacc ttgatggtca gca 23

<210> 62
<211> 29
<212> DNA
<213> Mycoplasma genitalium

<400> 62
atatcatata aagctctacc gttgttatc 29

<210> 63
<211> 18
<212> DNA
<213> Mycoplasma genitalium

<400> 63
actttgcaat cagaaggt 18

<210> 64
<211> 26

<212> DNA
<213> Mycoplasma genitalium

<400> 64
gtaatacata ggtcgcaagc gttatc 26

<210> 65
<211> 21
<212> DNA
<213> Mycoplasma genitalium

<400> 65
caccacctgt cactcggtta a 21

<210> 66
<211> 19
<212> DNA
<213> Mycoplasma genitalium

<400> 66
tagatactag ctgtcggag 19

<210> 67
<211> 19
<212> DNA
<213> Mycoplasma genitalium

<400> 67
acagagcgaa ccaagaatg 19

<210> 68
<211> 19
<212> DNA
<213> Mycoplasma genitalium

<400> 68
tagcctggat ctccttggt 19

<210> 69
<211> 21
<212> DNA
<213> Mycoplasma genitalium

<400> 69
aggaaactgc ttcctcagca g 21

**Description**

TECHNICAL FIELD OF THE INVENTION

The present invention relates to a highly sensitive method to detect a target nucleic acid in a sample. The present invention originates in the field of molecular biology and in particular in the field of PCR analysis.

The method of the invention finds a specific application in the field of microbiological diagnostics.

STATE OF THE ART

The polymerase chain reaction, commonly known as PCR, is one of the most widely available and broadly used methods for amplification and detection of target nucleic acid sequences.

PCR is a molecular biology technique that allows multiplication or amplification of nucleic acid fragments whose initial and terminal sequences are known. This technique was initially described by Saiki et al. 1985, Science 230:1350.

PCR amplification allows to rapidly obtain a large amount of a specific DNA sequence of interest *in vitro,* starting from extremely small quantities of genetic material.

Amplification of the target sequence involves the use of two oligonucleotide primers that hybridize to opposite strands, a polymerizing agent, typically an enzyme, and the target nucleic acid sequence. PCR technology involves a series of repetitive cycles of nucleic acid denaturation, primer annealing and extension of hybridized primers by the DNA polymerase enzyme in order to produce a large number of copies of a specific nucleic acid segment whose termini are defined by the 5' ends of the primers.

The choice of primers is crucial for successful PCR. Some factors that should be considered when designing primers are their length and GC content, moreover self-annealing sequences should be avoided.

In particular, the length and the sequence of primers affect their melting temperature (Tm between 50-65°C).

The Tm is the temperature at which half of the DNA molecules are in the double-helical state (double strand) and half are in the denatured single-stranded state. It is basically an index that allows to determine the temperature at which primers will be able to hybridize to their target sequences (annealing temperature, 5-6 °C bellow the Tm). Finally, the annealing temperature must be set in the instrument at values clearly below the Tm of the primers in order to allow hybridization, however, the values should not be too low, in order to prevent problems of non-specific hybridization.

Detection of PCR products is performed by two techniques: 1) electrophoretic analysis of the amplified product and 2) sequence-specific hybridization probes. In the former case, the PCR product is analyzed in agarose gel, which is basically a matrix functioning as "molecular sieve". Within the matrix, DNA molecules migrate under the influence of an electric field with a different speed depending on their size (hence on their molecular weight, MW): the amplified DNA (target DNA) will be thus visualized using appropriate DNA dyes (ethidium bromide). A successful amplification reaction shows a signal (typically a band) corresponding to a DNA molecule with MW consistent with the target sequence. In case of detection by hybridization probes, the PCR product is transferred, for example, to a membrane support, usually nitrocellulose, under denaturing conditions and is subsequently hybridized to labelled probes that are specific for the target sequence: a radioactive or colorimetric signal is obtained at the site of hybridization, thus where the amplified target sequence is present.

Real-Time PCR (RT-PCR) is a method for simultaneous amplification and quantification of DNA. DNA is amplified by chain reactions catalyzed by the DNA polymerase enzyme. DNA is quantified after each amplification round.

Therefore, it represents a particular version of PCR enabling quantitative determination of the starting DNA material by use of fluorescent DNA-intercalating dyes (eg, Syber Green) or oligonucleotides (termed probes) that fluoresce when hybridized to DNA and are specific for a particular target sequence within the amplified sequence (different technologies are available, such as Taqman, Molecular beacons, Scorpion).

RT-PCR has several advantages over traditional PCR: greater speed and specificity, eliminating post-PCR steps (agarose gel electrophoresis or probe hybridization on nitrocellulose filters), less contamination issues, simultaneous identification of multiple target sequences and ability to determine the amount of starting target DNA.

In addition, the use of detection probes is more accurate and reliable than intercalating dyes: indeed such probes are characterized by a specific sequence allowing highly selective hybridization to the target sequence, unlike intercalating agents interacting with any double-stranded DNA molecule. The concept of using probes with lower Tm than the primers used for the amplification, in order to prevent interference of the probe during the amplification reaction is disclosed e. g. in WO2006/044994, EP 1 942 196, WO 03/054233, WO 2007/149903.

In particular, probes based on the Taqman technology are oligonucleotides of length comprised between 20-30 nucleotides carrying a fluorescent dye attached to one end (REPORTER) while the other end carries a dye that "shields" the signal of the reporter (QUENCHER); therefore, when they are intact, these probes do not emit any signal. During the primer extension step, DNA polymerase synthesizes the nascent strand until reaches the sequence where the template strand is already hybridized to the probe. At this point the 5'→ 3' exonuclease activity of DNA polymerase starts to degrade the probe in order to continue strand synthesis. Reporter and quencher, previously placed in close proximity, are separated as result of degradation: the quencher can no longer exert its shielding action and thus the reporter emits a fluorescent signal that can be recorded by an appropriate detection system incorporated in the thermal cycler instrument. Clearly, fluorescence increases with each amplification cycle, depending on probe degradation rate. In fact, this will increase as the amplified material increases: the greater the amount of amplified DNA, the greater the amount of probe that pairs with the template strand, and the greater the number of probe molecules that will be degraded by DNA polymerase.

In a PCR experiment, DNA amplification follows an exponential trend which theoretically allows to relate the amount of amplified DNA to the amount of starting DNA. In practice, however, the increase follows an exponential trend only for a limited time corresponding to a certain range of thermal cycles.

The choice of primers and probes is obviously crucial for the success of RT-PCR: in addition to the above mentioned specific characteristics of primers, it is necessary to also consider the characteristics of fluorescent probes. These should ensure the highest possible specificity for target DNA, and at the same time they should have a melting temperature (hence an annealing temperature) higher than the primer (6-8°C higher) in order to remain paired to the target strand during the extension phase, which occurs at a temperature around 72°C. In this way they can be degraded by DNA polymerase, thus generating a fluorescent signal allowing Ct determination and accurate quantification.

However, the level of detection sensitivity and specificity for the target sequence that can be obtained with previously identified conventional detection methods does not always meet the analytical performance requirements. Such demand is particularly strongly felt for detection of certain specific targets, for instance in the field of microbiological diagnostics. More specifically, when considering some pathogens, for example bacterial pathogens such as C. trachomatis, N. gonorrheae and viral pathogens, there is a high demand for a technology capable of detecting and quantifying low levels of pathogens and/or in presence of high amounts of interfering substances in the test sample.

Therefore, one aim of the present invention is to provide a method for detecting a target nucleic acid sequence in a sample that is highly sensitive and allows detection even when the target sequence is present in low amounts or there are interfering substances in the sample to be analyzed.

Another aim of the present invention is to provide a system for analysis of a biological sample in homogeneous phase that allows amplification of a target sequence in a single reaction environment, thereby simplifying the process and reducing the risk of sample contamination associated with post-amplification handling.

SUMMARY OF THE INVENTION

According to a first aspect of the present invention, a method is provided for detection of a target nucleic acid sequence in a sample comprising
a step a) of amplification of the target nucleic acid sequence in presence of at least one primer and one probe, wherein the primer melting temperature (Tm) is at least 5°C higher, preferably 10°C higher and even more preferably 20°C higher than the melting temperature (Tm) of the probe and wherein said amplification occurs at a temperature above the Tm of said probe to allow hybridization of the primer to the target nucleic acid sequence essentially in the absence of substantial hybridization of said probe, and a step b) of amplification and detection by RT-PCR of the signal produced by hybridization of said probe with said of target nucleic acid sequence.

Additional features and embodiments of the method of the invention are set forth in the appended claims 2-10.

In particular, the method of the invention comprises

a step a) of pre-enrichment of the target nucleic acid sequence comprising an amplification reaction of the target nucleic acid sequence in presence of at least one primer and one probe, wherein the primer melting temperature (Tm) is at least 5°C higher, more preferably from 5 to 25°C higher than the probe melting temperature (Tm), said amplification being characterized in that it comprises an annealing step at a temperature higher, eg. 5 to 25°C higher, than the Tm of said probe, in order to allow hybridization of the primer to the target nucleic acid sequence essentially in the absence of probe hybridization,
b) a RT-PCR type amplification reaction comprising a step of annealing at a temperature lower, preferably at least 5°C lower, more preferably at least 10°C lower, even more preferably 5 to 25°C lower than the annealing temperature in step a) however close to the probe Tm in order to allow also hybridization of said probe to the amplification reaction mixture of step a).

Typically, conditions used in step b) of the method are such that both primer and probe can hybridize to their respective target sequences resulting in amplification of the target nucleic acid sequence and signal detection starting from the pre-enriched sample from step-a).

In this way, the method of the invention combines, in a single amplification protocol, a RT-PCR type amplification technology with an initial pre-enrichment step.

The combination of pre-enrichment and RT-PCR according to the present invention results in a striking increase of detection sensitivity of the technology, thus allowing detection of even very low amounts of target nucleic acid sequences in the starting biological samples.

In particular, the combination of step a) and step b) of the method of the invention allows detection and quantification of DNA extracted from a biological sample even in the presence of interfering substances in the sample.

According to another aspect, the present invention provides a highly sensitive method for amplification of a target nucleic acid sequence in a sample comprising

> a) a pre-enrichment step of the target nucleic acid sequence comprising

>> i) providing an amplification reaction mixture comprising the conveniently denatured target nucleic acid sequence, at least one primer, typically two primers, one probe, a polymerization inducing agent, deoxyribonucleoside triphosphates (dNTPs), wherein the primer has a Tm at least 5°C higher, preferably at least 10°C higher, than the Tm of said probe;
>> ii) subjecting said amplification reaction mixture to multiple repeated thermal cycles comprising primer hybridization to complementary sequences of the target nucleic acid sequence and extension of said sequences complementary to the target sequence by the polymerization inducing agent, said pre-enrichment step being characterized in that the annealing occurs at a temperature higher, preferably at least 5°C higher, more preferably at least 10°C higher than the Tm of said probe in order to allow primer hybridization to the nucleic acid sequence in the absence of substantial hybridization of said probe,

> b) a RT-PCR type amplification reaction comprising an hybridization (annealing) step at a temperature preferably 5°C lower, more preferably at least 10°C lower, than the temperature of the annealing step during pre-enrichment, however close to probe Tm in order to allow also probe hybridization to the amplification reaction mixture of step a).

In one form of realization, the method of the invention includes the following two steps:

> step a) of pre-enrichment, including a higher annealing T, preferably at least 5°C higher, preferably 10°C higher than the probe Tm, wherein only the primers can hybridize to the target sequence and wherein amplification of target DNA occurs with high specificity and efficiency,
> a step b) by RT-PCR comprising a lower annealing T preferably at least 5°C lower, more preferably at least 10°C lower than the temperature in step a) and however close (preferably +/- 5°C, more preferably +/-3°C) to probe Tm, wherein primer and probe can hybridize to their respective target sequences and wherein DNA amplification and simultaneous signal detection from the sample pre-enriched in step a) take place.

It is advantageous that the combination of steps a) and b) allows very efficient detection of the target nucleic acid sequence, providing greater sensitivity since the $C_T$ appears earlier than in a conventional RT-PCR.

Typically, the starting material of the method of the invention is DNA or cDNA extracted from a biological sample.

A preliminary step of the method of the invention involves extraction of DNA (viral or bacterial) or RNA (viral) starting from different types of biological specimens such as blood, saliva, tissues, urine, typically from swabs, samples, biopsies. This extraction is performed by conventional methods for isolation and purification of DNA or RNA of pathogens that may be present in the sample.

After DNA extraction, the detection of DNA (RNA) of the target microorganism is carried out by the method of the invention that in one aspect involves amplification of DNA or cDNA (obtained by prior reverse transcription of RNA) according to the previously described step a), followed by RT-PCR according to the step b), using a RealTime instrument that allows signal detection.

For the purpose of the invention the term target sequence or target nucleic acid sequence refers to the oligonucleotide region to be amplified or detected, or both. Typically, the target nucleic acid sequence resides between the two primer sequences used for amplification.

DETAILED DESCRIPTION OF THE INVENTION

According to one realization, the detection method of the invention involves the use of a primer with a Tm at least 5°C higher, preferably 10°C higher, than the Tm of the probe. This feature allows annealing of primers only to their target sequence during the step a) of pre-enrichment (amplification), while the probe, with a lower Tm, cannot anneal because the temperature that is set in the step a) does not allow it to anneal. In the following step b), the temperature during the annealing step is lowered to a value that allows also the probe to work together with primers, thus starting a RealTime PCR reaction. Under these conditions, primers are under permissive conditions during the annealing step, but the sample is already enriched in target DNA from the previous step a) during which only the primers worked with high specificity (a high Tm allows high annealing temperatures).

In the step a) of pre-enrichment, primers are conveniently allowed to work with higher effectiveness on the sequence to be amplified, thus resulting in increased specificity. In the step b), when probes start functioning upon lowering the annealing temperature, amplification occurs from material that has been already selectively enriched in target DNA, thus allowing a crucial improvement of performance of the primer/probe system.

Typically, two primers are used for the step a) of amplification, and their Tm is preferably the same.

Typically, the method of the invention can be applied to detect the presence of a microbial or viral agent in a sample.

For the purpose of the invention, the term sample refers to any substance containing or considered to contain the nucleic acid, and includes a tissue sample or a fluid sample isolated from an individual, including without being limited to: plasma, serum, skin, urine, blood cells, cerebrospinal fluid, lymph, tumor cells, and also samples from in vitro cell cultures.

According to one form of realization, the method of the invention is applied to detect in a biological sample the presence of a strain of a microorganism, as for instance Chlamydia Trachomatis, Hepatitis C virus, Hepatitis B virus, Toxoplasma gondii, Hepes simplex virus, Varicella zoster, Epstein-Barr virus, Citomegalovirus, Enterovirus, BK virus, JC virus, Chlamydia phage, Clostridium difficile, and Mycoplasma genitalium.

In a preferred aspect of the present invention the microorganism is selected from the group consisting of Chlamydia Trachomatis, Clostridium difficile, and Mycoplasma genitalium.

According to one form of realization, for detection of a microbiological agent, in particular Chlamydia trachomatis, primers are used selected from

TGGCGATATTTGGGCATCCGAGTAAC (SEQ ID N°1),
TACGCTCAAATCCAGCGGGTATTAACCG (SEQ ID N°2)

and their homologs with a degree of homology equal to or greater than 80%.

The method of the invention has the advantage to enable simultaneous amplification and detection of target nucleic acids in a single reaction environment in which both steps a) and b) take place, thus avoiding transfer and handling of samples and thereby improving analytical accuracy and speed.

For the purpose of the invention, the term amplification typically refers to an exponential increase of the target nucleic acid and includes both linear and exponential increase of the number of a target nucleic acid sequence. A typical amplification reaction is for instance described in patent EP 201 184.

The term PCR amplification refers to a process for amplification of nucleic acids involving the use of two oligonucleotide primers, a polymerizing agent, a target nucleic acid template and subsequent cycles of nucleic acid denaturation and primer hybridization and extension to produce a large number of copies of a nucleic acid segment. PCR methods are described for example in U.S. 4,683,202 ,

The term amplification reaction mixture refers to an aqueous solution comprising the various reagents used to amplify the target nucleic acid. These components include one or more amplification primers, target nucleic acids, nucleoside triphosphates, polymerization-inducing agents, salts, buffers.

For the purpose of the invention, the polymerization inducing agent is typically an enzyme such as polymerase that catalyzes polymerization of nucleoside triphosphates. Typical polymerases include for example E. coli polymerase, T4 DNA polymerase, and preferably thermostable polymerases, i.e. polymerases which are relatively heat-stable compared, for example, to nucleotide polymerases from E. Coli. For example, Taq (Thermus aquaticus) polymerases can be used, as described in U.S. 4,889,818, also in a conventional PCR as described in Saiki et al. 1988 Science 239:487.

The term nucleic acid is meant to comprise any DNA or RNA polynucleotide, genomic, cDNA, of natural, synthetic or semi-synthetic origin.

The term primer comprises one or more suitable primers in the context of the method of the invention, and refers to oligonucleotides capable of acting as point of initiation of synthesis along a complementary strand, under reaction conditions in which synthesis of an extension product complementary to the target nucleic acid strand is catalyzed.

These reaction conditions typically include the presence of 4 different deoxyribonucleotides triphosphates and an enzyme such as DNA polymerase or reverse transcriptase in an appropriate buffer at an appropriate temperature.

The term melting temperature Tm refers to the temperature at which 50% of base pairs are separated.

For the purpose of the invention, the term probe refers to a labeled oligonucleotide which forms a duplex structure with a sequence in the target nucleic acid, due to complementarity of at least one sequence of the probe to one sequence in the target region. Preferably the probe does not contain a sequence complementary to sequence(s) used to prime the polymerase chain reaction. Typically the 3' end of the probe is blocked to prevent incorporation of the probe itself in a primer extension product. Blocking can be obtained by conventional techniques, for example using non-complementary bases or by addition of a chemical moiety such as biotin or a phosphate group to the 3' hydroxyl of the last nucleotide.

The term labelled refers to any atom or molecule that can be used to provide a detectable signal, that is preferably quantifiable, and that can be attached to a nucleic acid or a protein. Labels may provide signals detectable by fluorescence, radioactivity, colorimetry, gravimetry, X-ray diffraction or absorption, magnetism, enzymatic activity and the like.

Typically, the labelled probe is first hybridized to a complementary nucleic acid before nucleic acid polymerase encounters the duplex region, thereby allowing the 5' to 3' nuclease activity to cleave and release oligonucleotide fragments that are labeled and detectable.

BRIEF DESCRIPTION OF THE DRAWINGS

The present invention can be fully understood by reading the following detailed description and the following examples with reference to the accompanying drawings where:

Figure 1 shows a growth curve of the nucleic acid during step b),
Figure 2 shows the change of the threshold cycle (Ct) that occurs in the step B in one form of realization of the invention.

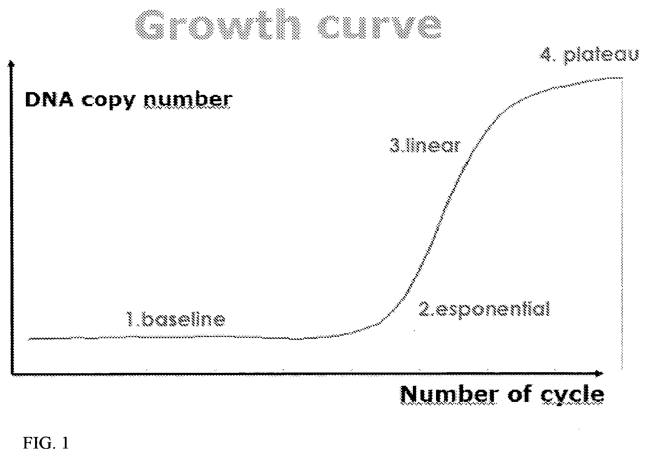

In one form of realization of the invention, the actual DNA growth curve follows a profile as shown in Figure 1, due to a number of factors including decreased polymerase activity and number of available primers over time.

A steady state is reached during the exponential (log) phase, which basically lacks any limiting factor; the amount of amplified DNA is directly correlated with the amount of starting target DNA. Highly reproducible measurements are indeed observed between different replicates, therefore this represents the best phase for accurate quantification.

It is possible to establish a relationship between the amount of amplified DNA and the amount of starting target DNA that applies to the exponential phase and can be expressed by the following equation:

$$Y = X \, (1+E)n$$

where:

Y = amount of PCR amplified DNA
X = amount of target DNA prior to PCR amplification
E = amplification efficiency
n = number of cycles

It can be inferred from this equation that a 10-fold greater amount of DNA is obtained every 3.32 cycles, i.e. 1 log difference.

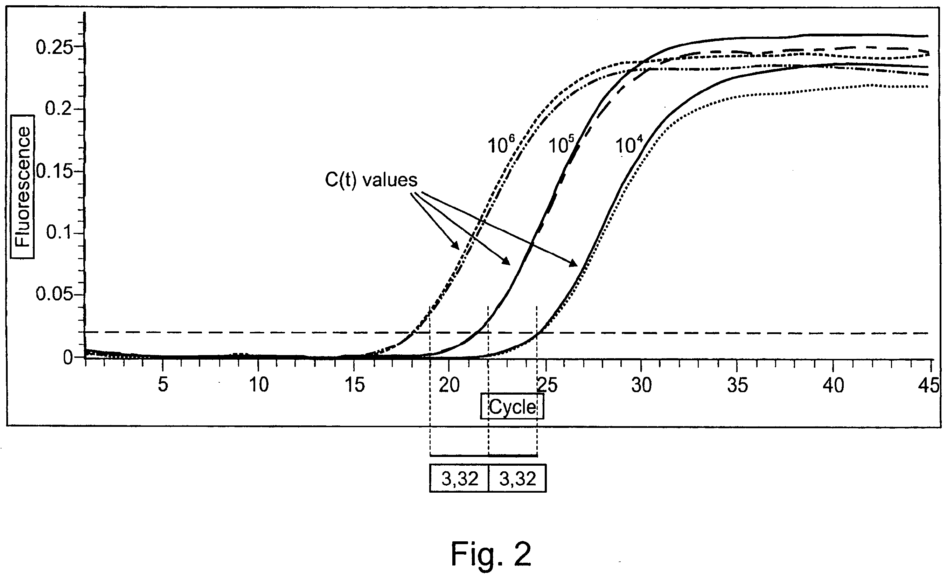

A key parameter for quantification in RT-PCR is the threshold cycle (Ct), which is defined as the point of the amplification reaction during which the fluorescence signal rises significantly above the background signal. As shown in Figure 2, CT is related to the starting amount of target DNA. Because CT is generally within the exponential phase, it is possible to establish a linear relationship between the log of the starting amount of target DNA and the corresponding CT during RT-PCR: the greater the amount of target DNA, the lesser the number of cycles needed to obtain a significant signal over background (CT). By preparing serial dilutions of a sample of known concentration it is possible to make a calibration curve relating the log of known amounts of starting DNA to their CT expressed during the amplification reactions, and to use such curve to quantify the target sample.

In this context, an essential aspect is played by the choice of the characteristics of the primer and the probe.

In the context of the invention, primers preferably have a Tm that is 10°C higher, more preferably 20°C higher than the Tm of the probe. This feature is essential for proper functioning of the step b) that comprises a RT-PCR reaction. Under these reaction conditions, the probe is able to efficiently hybridize to the target sequence during the step of strand synthesis and therefore can be degraded by the exonuclease activity of the polymerizing agent, typically a DNA polymerase, thus generating a detectable fluorescent signal. Moreover, if the temperature of the extension step would be lower than the probe Tm, hybridization of the probe would be too permissive, thus decreasing specificity. In these conditions it is possible to realize the method of the invention wherein the two phases a) and b), pre-enrichment and RT-PCR, allow concomitant amplification and detection of the target nucleic acid sequence.

In particular, in step a) only primers can hybridize to their target nucleic acid sequence while the probe, with very low Tm, cannot hybridize because the process temperature is too high. Subsequently, owing to the appropriate amplification protocol that is preset in the instrument, the temperature decreases significantly during the annealing step, allowing the probe to work together with primers, thus starting the classical RealTime process. Under these conditions, primers are under permissive conditions during the annealing step, but the sample is already enriched in target DNA from the previous step a) during which only primers worked with high specificity (high Tm allows high annealing temperatures). Under these conditions, the first step allows primers to work more efficiently on the sequence to be amplified, thus increasing specificity; in the second phase, when probes enter into play at a lower annealing temperature, amplification occurs starting from the material already selectively enriched in target DNA, with significant improvement of performance of the primer/probe system.

From an analytical point of view, the application of the method of the invention results in a clear reduction of Ct: signal detection occurs starting from a target DNA sample that is already enriched from a series of previous cycles, during which primers worked under conditions of high specificity.

This feature is extremely advantageous for the analysis of biological samples which may contain a low amount of starting target DNA and/or high amounts of interfering substances, for example after a relatively inefficient extraction procedure.

Typically these samples have very high Ct in a standard RT-PCR, giving quantification problems, or do not reach a Ct during the whole amplification process, hence do not yield a detectable signal, thus producing a negative result.

The method of the invention makes possible to overcome these problems by using a probe Tm that is lower than primer Tm, in combination with an appropriate thermal profile, thus allowing selective enrichment of the target in the initial step under conditions of high specificity, followed by production and detection of the fluorescence signal.

It is advantageous that a combination of the pre-enrichment step a) with the step b), according to the invention, turns out to be more efficient than conventional techniques considered individually. The advantages of the method of the invention are even more evident for samples yielding a very high Ct during RT-PCR, when the system is already in the final stage of the amplification process and therefore tends to lose linearity; when analyzed by the new technology, such samples are characterized by a clearly lower Ct. This decrease is not solely due to the number of pre-enrichment cycles carried out prior to the second step of amplification; the overall Ct is lower than in a conventional RT-PCR even considering the amplification cycles in the first step. This is due to the effective and specific action of primers with selected Tm used during the pre-enrichment step, enabling better functioning of the primer/probe system in the second step, thereby reaching the Ct more quickly, ultimately resulting in increased sensitivity of the system. Obviously, this feature is extremely useful also for samples that failed to produce a detectable signal (CT) with a conventional amplification protocol in a standard RT-PCR, also due to the presence of large amounts of interfering substances. A simple extension of the RT-PCR amplification protocol would not solve the problem in this case: a detectable signal would be obtained in a too late stage of the amplification protocol; the system would fail to ensure a sufficiently reliable exponential phase (hence linearity). Moreover, an excessive length of the amplification protocol increases the probability of appearance of non-specific signal. Using the method of the invention, the primer/probe system works more favourably because of the pre-enrichment step, thereby ensuring that the Ct appears under conditions of linearity of the system, thus rendering quantification a more reliable and reproducible task and, above all, increasing the performance in terms of sensitivity.

The method of the invention is thus particularly suitable for applications where the search for the target microorganism is particularly difficult due to a low level of the sample and/or presence of interfering substances.

Typically the method of the invention finds a suitable application in the field of microbiological diagnostics. In this context, a particular application consists in designing a probe that identifies a target sequence shared by a series of microorganisms, typically different strains of a single bacterial species, in order to detect with a single probe (and a single fluorescence signal) a whole family of target organisms, i.e. different strains of the same species.

In this case, once appropriate primers are chosen, the method of the invention makes possible to carry out a single amplification protocol even with a probe whose Tm is lower than the permissive values for RT-PCR, for instance 58-60°C.

EXAMPLES OF THE INVENTION

The following examples are provided only to illustrate the present invention and should not be intended as limiting the scope of protection, as evidenced by the included claims.

**EXAMPLE 1**

The method according to an embodiment of the invention was applied to seek and detect DNA from Chlamydia trachomatis in clinical specimens based on DNA extracts from biological matrices such as urine and urethral swabs, and was compared with the standard RT-PCR method.

Results were then compared with a conventional ProbeTec Beckton Dickinson PCR reference system.

Extracts were obtained starting from the lysate made by the automatic system (ProbeTec). Because most of the material was needed for the subsequent protocol of analysis with the ProbeTec technology (reference system), a limited amount of material was available for the other applications (EuroClone RT-PCR method and method of the invention). In fact, only 1/10 of the starting ProbeTec lysate was subjected to a further extraction step (DuplicaPrep-EuroClone automated system) for analysis with the EuroClone systems (see table "Experimental Protocol").

Materials and methods

For realization of the method, the primers and one probe were selected in order to perform a first step a) of amplification where only primers work (high-annealing temperature) and a second step b) where also the Probe works (low annealing temperature).

Selected oligonucleotides had a high melting temperature (Tm) in the case of primers (Fwd primer Tm: 66.5°C; Reverse Primer Tm: 68.4°C) and a lower Tm in the case of the probe (probe Tm: 45.4°C).

The following oligonucleotide sequences were selected for Chlamydia trachomatis:

Primer Fwd: 5'-TGGCGATATTTGGGCATCCGAGTAAC, (SEQ ID NO.1)
Primer Rev: 5'-TACGCTCAAATCCAGCGGGTATTAACCG, (SEQ ID NO.2)
Sonda: 5'-FAM-ACCGAAAGGTCCTAAGA-BHQ1, (SEQ ID NO.3)

Sequences for internal control of amplification of are:

primer Fwd:5'-ACAGAGCGAACCAAGAATG, (SEQ ID NO.4)
Primer reverse: 5'-TAGCCTGGATCTCCTTGGT, (SEQ ID NO.5)
Probe: 5'-HEX-AGGAAACTGCTT CCTCAGCAG-BHQ1. (SEQ ID NO.6)

The primers employed for the method according to the invention contain the same sequence as those for the standard RT-PCR used for comparison, in addition 5 nucleotides were added to the 5' end + one nucleotide was added to the 3' end of the FWD primer, and 5 nucleotides were added to 5' and 3' ends of the REV primer.

Lengthening primers helped to increase the difference between primer Tm and probe Tm.

The reaction mixture is as follows:

| | |
|---|---|
| Primer Fwd Chlamydia | 0.3 $\mu$M (final concentration) |
| Primer Rev. Chlamydia | 0.3 $\mu$M (final concentration) |
| Specific Probe Chlamydia | 0.4 $\mu$M (final concentration) |
| Internal Cont. Fwd Primer | 0.2 $\mu$M (final concentration) |
| Internal Cont. Rev. Primer | 0.2 $\mu$M (final concentration) |
| Internal Control Probe | 0.2 $\mu$M (final concentration) |
| Glycerol | 2% (final concentration) |
| Reaction Buffer | 10X 1X (final concentration) |
| dNTPs 25mM | 0.32mM (final concentration) |
| MgCl2 50mM | 3.2mM (final concentration) |
| Taq polymerase | 0.12U/$\mu$l (final concentration) |
| H2O as needed to | 20 $\mu$l |

Dispense 20$\mu$l of Mix in PCR tubes, one for each test sample, to each tube add 1$\mu$l of internal control for amplification and 5 $\mu$l of DNA extract.

| | |
|---|---|
| Amplification Mix | 20 $\mu$l |
| Internal Control | 1 $\mu$l |
| Extracted DNA | 5 $\mu$l |
| Total volume | 26 $\mu$l |

The samples prepared as described above are applied to the SmartCycler (Cepheid) real-time instrument that is programmed to execute the following amplification protocol:

> 95 °C for 5 minutes
> (95°C for 15 seconds, 68°C for 60 seconds) x 10 cycles signal acquisition OFF
> (95°C for 15 seconds, 54°C for 60 seconds) x 50 cycles signal acquisition ON
> Select Fluorescence channels for FAM (specific probe) and HEX (internal control)

RESULTS:

Diagnostic sensitivity and specificity

Table 1 shows the results obtained with the method based on a conventional RT-PCR technology and the results obtained with the method of the invention.

Results expressed as Ct values are compared each other and with the reference method (ProbTec). As it can be easily seen, 6 negative samples were obtained out of 48 samples using the RT-PCR method; of these 6 negative samples only 2 scored negative also according to the reference method, while the remaining 4 samples scored positive (i.e. they were false negatives). Using the new technology, the results fully agree with the reference method: 2 negatives out of a total of 48 clinical samples.

> Method of the invention: sensitivity 100%, specificity 100%
> Conventional RT-PCR: sensitivity 83%, specificity 100%

The 4 false negatives (obtained by standard RT-PCR) scored positive with the technology of the invention and with the reference method.

This confirms the above mentioned characteristics of the new technology. These 4 samples are in fact characterized by a very small amount of starting target DNA and/or presence of interfering substances which make them difficult to be used for detection by a standard RT-PCR method. In contrast, by using the method of the invention it is possible to obtain a clearly detectable signal (although at a high Ct) and thereby increase the diagnostic performance in terms of sensitivity. Moreover, it should be emphasized the decrease of Ct obtained with the method of the invention compared to the RT-PCR method. In particular, it can be observed that some samples showing a rather low Ct using the classical RT-PCR method (<30° cycle, ie. samples 2-3-22-37), show a Ct value with the new technology that is about 10 cycles lower compared to classical RT-PCR. This difference actually corresponds to 10 cycles carried out in the first step of sample pre-enrichment that was set according to the new technology. Therefore using the new technology may not result in a clear advantage for this kind of samples containing rather large amounts of starting target DNA. In contrast, when considering other samples showing a high Ct with the standard RT-PCR technology (e.g. 4-8-11-14-23-35-47-48), there is a more clear difference from the Ct values obtained with the new technology, in some cases more than 20 cycles. This difference exceeding the number of cycles in the pre-enrichment step of the new technology represents an unquestionable advantage: it is clear that the sample enrichment step (1st phase, analogous to a classical PCR) renders the subsequent RT-PCR step more efficient thereby improving the overall performance of the system in terms of sensitivity.

Moreover, this result is not simply due to the fact that primers used in the method of the invention ensure better performance compared to those in standard RT-PCR.

In fact, a comparison of the results from standard RT-PCR with those from the new technology, with regard to samples with a low Ct, shows an actual difference of 10 amplification cycles (corresponding to 10 cycles in the pre-enrichment of step a) without a net increase of system performance for this type of samples.

Table 1

| Samples | Reference | New technology | Current method (RT-PCR) |
|---|---|---|---|
| | | **CT** | **CT** |
| **1** | **POS** | **27.86** | **NEG** |
| **2** | **POS** | **16.16** | **25.31** |
| **3** | **POS** | **16.70** | **25.86** |
| **4** | **POS** | **18.42** | **39.41** |
| **5** | **NEG** | **NEG** | **NEG** |

(continued)

| Samples | Reference | New technology | Current method (RT-PCR) |
|---|---|---|---|
| | | CT | CT |
| 6 | POS | 22.16 | 38.79 |
| 7 | POS | 19.59 | 38.23 |
| 8 | POS | 21.42 | 40.75 |
| 9 | POS | 16.82 | 36.49 |
| 10 | POS | 16.34 | 31.74 |
| 11 | POS | 24.97 | 43.56 |
| 12 | POS | 16.39 | 26.59 |
| 13 | POS | 20.70 | 32.86 |
| 14 | POS | 25.20 | 43.83 |
| 15 | POS | 33.80 | NEG |
| 16 | POS | 20.24 | 30.50 |
| 17 | POS | 19.39 | 29.56 |
| 18 | POS | 22.67 | 36.91 |
| 19 | POS | 22.95 | 38.32 |
| 20 | POS | 23.59 | 34.16 |
| 21 | POS | 25.37 | 42.31 |
| 22 | POS | 18.06 | 27.49 |
| 23 | POS | 24.63 | 43.98 |
| 24 | POS | 24.24 | 42.48 |
| 25 | POS | 16.73 | 27.51 |
| 26 | POS | 22.24 | 39.78 |
| 27 | POS | 21.47 | 34.98 |
| 28 | POS | 20.15 | 35.39 |
| 29 | POS | 17.93 | 28.36 |
| 30 | POS | 37.77 | 47.30 |
| 31 | POS | 23.37 | 38.31 |
| 32 | POS | 22.83 | 34.33 |
| 33 | POS | 31.79 | NEG |
| 34 | POS | 16.10 | 27.69 |
| 35 | POS | 21.97 | 46.73 |
| 36 | POS | 24.86 | 44.69 |
| 37 | POS | 14.10 | 24.43 |
| 38 | NEG | NEG | NEG |
| 39 | POS | 16.99 | 28.37 |
| 40 | POS | 23.89 | 42.86 |
| 41 | POS | 28.09 | NEG |
| 42 | POS | 22.09 | 33.82 |

(continued)

| Samples | Reference | New technology | Current method (RT-PCR) |
|---------|-----------|----------------|-------------------------|
|         |           | CT             | CT                      |
| 43      | POS       | 23.97          | 42.82                   |
| 44      | POS       | 16.65          | 35.82                   |
| 45      | POS       | 19.32          | 37.34                   |
| 46      | POS       | 18.05          | 37.30                   |
| 47      | POS       | 22.64          | 47.17                   |
| 48      | POS       | 25.41          | 44.14                   |

Analytical specificity:

Table 2 summarizes the results obtained from samples that were negative for Chlamydia t. and positive for other pathogens, in order to verify the specificity of the method based on the new technology:

Table 2

|                         | CT             |      |
|-------------------------|----------------|------|
|                         | Specific Probe | I.C. |
| Samples                 |                |      |
| E.Coli                  | --             | 27.93 |
| Legionella              | --             | 30.55 |
| Salmonella              | --             | 30.28 |
| Mycoplasma Genitalium   | --             | 31.09 |
| Mycoplasma Hominis      | --             | 28.86 |
| Clamidia Pneumoniae     | --             | 30.40 |
| Neisseria Gonorrhoeae   | --             | 30.16 |
| Ureaplasma Urealyticum  | --             | 31.02 |
| Trichomonas Vaginalis   | --             | 29.09 |
| Candida Albicans        | --             | 20.43 |
| Listeria Monocitogen es | --             | 31.03 |
| Aspergillus             | --             | 30.15 |
| G7                      | --             | 17.21 |
| G19                     | --             | 18.27 |
|                         | Specific Probe | C.I. |
| Samples                 |                |      |
| G20                     | --             | 21.15 |
| G45                     | --             | 20.31 |
| G46                     | --             | 22.16 |
| 26TV                    | --             | 19.07 |
| 5/10                    | --             | 20.52 |
| 4/09                    | --             | 20.27 |
| 7/10                    | --             | 22.69 |

(continued)

|  | Specific Probe | C.I. |
|---|---|---|
| Samples |  |  |
| 53/07 | -- | 22.49 |
| 51/07 | -- | 20.05 |
| 3/10 | -- | 21.07 |
| C.P. | 18.39 | 32.04 |
| Neg | -- | 32.56 |
| Blank | -- | -- |

Controls (Positive and Negative controls for the Test)
Negative samples (Genomic samples positive for various pathologies)
Negative-samples (samples highly positive for Neisseria gonorrhoeae)
Negative samples (samples of human genomic DNA)
Result: 100% specificity

Analytical Sensitivity:

In this test, a genomic sample of Chlamydia trachomatis, quantified at 10e4 copies/uL (ABI Advanced Biotechnologies Inc.), was tested at different dilutions in order to determine test sensitivity.

## Table 3

|  | CT | |
|---|---|---|
|  | Specific Probe | C.I. |
| Samples |  |  |
| ABI 10000 copies/reaction | 14.87 | -- |
| ABI 1000 copies/reaction | 18.57 | -- |
| ABI 100 copies/reaction | 21.04 | -- |
| ABI 10 copies/reaction | 24.69 | 27.56 |
| ABI 1 copies/reaction | 28.19 | 29.95 |
| ABI 0,1 copies/reaction | 30.52 | 28.33 |
| ABI 0,01 copies/reaction | -- | 29.36 |
| CP | 25.26 | -- |
| Neg | -- | 32.39 |

The sensitivity of this new technology on genomic samples (hence not on real clinical samples, and devoid of any possible interference) is comparable to that of a standard RT-PCR. Thus the method according to the invention is able to improve performance in terms of sensitivity on real clinical samples, where the first step of sample pre-enrichment actually allows RT-PCR to work more efficiently in the subsequent step, making the system more robust and improving performance in diagnostic sensitivity.

Reproducibility:

In this test, the reproducibility of the sensitivity limit of the assay was tested, thus a genomic sample with a known concentration of 1 copy/reaction (ABI Advanced Biotechnologies Inc.) was replicated 20 times in the same test.

Table 4

| | CT | |
| | Specific Probe | C.I. |
| --- | --- | --- |
| Samples | | |
| ABI 10 copies/reaction | 25.35 | 28.59 |
| ABI 1 copy | 28.06 | 29.75 |
| ABI 1 copy | 29.31 | 28.32 |
| ABI 1 copy | 28.35 | 28.77 |
| ABI 1 copy | 30.30 | 30.57 |
| ABI 1 copy | 28.54 | 28.23 |
| ABI 1 copy | 2943 | 28.66 |
| ABI 1 copy | 28.54 | 28.63 |
| ABI 1 copy | 28.72 | 29.16 |
| ABI 1 copy | 27.78 | 28.12 |
| ABI 1copy | 27.22 | 26.80 |
| ABI 1copy | 27.54 | 28.82 |
| | CT | |
| | Specific Probe | C.I. |
| Samples | | |
| ABI 1 copy | -- | -- |
| ABI 1 copy | 29.92 | 29.03 |
| ABI 1 copy | 28.41 | 29.09 |
| ABI 1 copy | 30.41 | 28.44 |
| ABI 1 copy | 29.01 | 28.75 |
| ABI 1 copy | 28.25 | 28.11 |
| ABI 1 copy | 28.51 | 29.22 |
| ABI 1 copy | 28.66 | 28.31 |
| ABI 1copy | 29.74 | 29.33 |
| CP | 18.62 | -- |
| Neg | -- | 27.56 |

Result: 95% reproducibility

**EXAMPLE 2**

Value of the step a) of pre-enrichment of the method of the invention.

To test the effectiveness of the sample pre-enrichment phase, bacterial DNA from C. trachomatis was sought in clinical samples consisting of DNA extracts from two biological matrices (urine and urethral swabs); this search was carried out by the method of the invention and was compared with a similar method without the pre-enrichment step (ie. only the RT-PCR step was carried out, using the same primers and probes as with the new technology).

These samples were treated identically to those described above. Extraction was with an automated system (ProbeTec) and also in this case there was a limited amount of residual material available for test applications (method of the invention vs similar method lacking the pre-enrichment step). In fact, only 1/10 of the starting ProbeTec extract was subjected to a further extraction step (DuplicaPrep-EuroClone automated system) for analysis with the test systems.

Table 5

| SAMPLE | REFERENCE (PROBETEC) | Realization of the invention without step 1 (Ct) | Realization of the invention step 1 + step 2 (Ct) |
|---|---|---|---|
| 19 | pos | 34.16 | 22.96 |
| 23 | pos | 31.31 | 21.44 |
| 26 | pos | 29.15 | 18.68 |
| 27 | pos | Neg | 24.54 |
| 28 | pos | Neg | 24.81 |
| 29 | pos | 31.73 | 21.98 |
| 32 | pos | 33.99 | 27.89 |
| 33 | pos | Neg | 33.47 |
| 57 | pos | Neg | 32.64 |
| 67 | pos | 28.71 | 19.04 |
| 77 | pos | Neg | 20.83 |
| 95 | pos | Neg | 31.79 |
| 97 | pos | Neg | 26.53 |
| 109 | pos | Neg | 33.89 |

Results clearly show that the step a) of pre-enrichment of the method is crucial for the functioning of the new technology. In fact it can be seen that, in a series of samples, the method of the invention lacking the pre-enrichment ste (essentially a standard RT-PCR) is unable to provide any detectable signal, whereas the same samples score positive by using an embodiment of the method (1st pre-enrichment step, 2nd RT-PCR ste). These results clearly demonstrate the combined effect of the two steps a) and b) of the technology of the invention. If the role of first step were limited to providing an advantage on the number of cycles, one would expect a Ct equal to that seen with the new technology + roughly 10 cycles, for samples that scored negative. In reality, even in the case of those samples characterized by a high Ct (low amount of starting target DNA and/or presence of contaminants) there was a combined effect of the pre-enrichment ste with the second RT-PCR step, resulting in net increase of the performance of the embodiment according to the invention.

**EXAMPLE 3**

COMPARISON OF STANDARD RT-PCR WITH THE NEW TECHNOLOGY ACCORDING TO THE PRESENT INVENTION

The advanced technology was used for detection of C. difficile DNA in clinical stool samples. Such samples were analyzed using a kit based on standard RT-PCR technology (developed by EuroClone) and with a kit based on the new technology. Results were compared to a conventional reference system (BD Gene Ohm).

Materials and methods

Samples analyzed with the Euroclone kit (classical RT-PCR and new technology) were extracted with the DuplicaPrep automated DNA extraction system according to instructions described in the instruction manual of the instrument.

For realization of the test, primers and probes were selected in order to perform a first step a) of amplification, where only primers work (high-annealing temperature), and a second step b) where also the Probe works (low annealing temperature).

The following oligonucleotide sequences were selected for two target genes of C. difficile (Toxa and ToxB):

C.D.TA Fwd:5'-CAATGGGCTGATATTAATGCAGAAT(SEQ ID NO.55) 57.7
C.D.TA Rev: 5'-TCATCTATTGTARGTACTGTAGGTTTATTG(SEQ ID NO.56) 54.9
C.D.TA Probe: 5'-Fam-ACAGCTACTAGAGGAAGAGAT-BHQ1 (SEQ ID NO.57) 46.5
C.D.TB Fwd1:5'-TGAGGACATATCAGAGACTGATGAGG(SEQ ID NO.58) 57.1
C.D.TB Rev1: 5'-TAGCATATTCAGAGAATATTGYYTTTTCAG(SEQ ID NO.59) 56.2
C.D.TB Probe1: 5'-Fam-CTGGAGAATCTATATTTGTAGAA-BHQ1 (SEQ ID NO.60) 45.7

The reaction mixture is shown below in Table 6::

| Primer mix | | | | | | Samples | Run |
|---|---|---|---|---|---|---|---|
| Initial conc. | | Reagent | Final Conc. | | Lot Vol (ul) | 1 | 10 |
| 100 | uM | Primer Fwd T.A. | 0.16 | uM | 0.04 | 0.04 | 0.4 |
| 100 | uM | Primer Rev T.A. | 0.16 | uM | 0.04 | 0.04 | 0.4 |
| 100 | uM | Spec. Probe T.A. | 0.16 | uM | 0.04 | 0.04 | 0.4 |
| 100 | uM | Primer Fwd T.B. | 0.16 | uM | 0.04 | 0.04 | 0.4 |
| 100 | uM | Primer Rev T.B. | 0.16 | uM | 0.04 | 0.04 | 0.4 |
| 100 | uM | Spec. Probe T.B. | 0.16 | uM | 0.04 | 0.04 | 0.4 |
| 100 | uM | Primer Fwd C.I. | 0.16 | uM | 0.04 | 0.04 | 0.4 |
| 100 | uM | Primer Rev C.I. | 0.16 | uM | 0.04 | 0.04 | 0.4 |
| 100 | uM | Spec. Probe C.I. | 0.16 | uM | 0.04 | 0.04 | 0.4 |
| 10 | % | Glycerol | 1.2 | % | 3 | 3 | 30 |
| | | H2O | as needed | | 7 | 7 | 66.4 |
| | | | Final volume | | | 10 | 10 | 100 |

| Reaction master mix | | | Samples | |
|---|---|---|---|---|
| Reagent | Final Conc. | Lot Vol (ul) | 350 | |
| 2x Master Mix probes | 1:1 | smar taq 03U/ul | 10 | 3500 |
| 2x Primer mix | 1:1 | see above | 10 | 3500 |
| | Final total volume | 20 | 7000 | |
| | DNA template | 5 | | |
| | Internal Control | 1 | | |

The samples prepared as described above are applied to the SmartCycler (Cepheid) real-time instrument that is programmed to execute the following amplification protocol:

**Thermal profile: 95°C for 5 min.**
**(95°C for 15 sec., 63°C for 1 min.)x 15 cycles**
**(95°C for 15 sec., 53°C for 1 min.)x 45 cycles**

Results

## LEGEND

| | |
|---|---|
| | EC POS/BD POS |
| | EC POS/BD NEG |
| | EC NEG/BD POS |
| | EC NEG/BD NEG |
| | INHIBITED |
| | NOT CALCULATED |

Results are shown in Table 7.

Table 7

| Samples | BD GeneOhm | EuroClone RT-PCR | EuroClone New technology |
|---|---|---|---|
| 1 | Pos | Pos | Pos |
| 2 | Pos | Neg | Pos |
| 3 | Pos | Pos | Pos |
| 4 | Pos | Neg | Pos |
| 5 | Pos | Neg | Pos |
| 6 | Pos | Neg | Pos |
| 7 | Pos | Pos | Pos |
| 8 | Pos | Neg | Pos |
| 9 | Pos | Pos | Pos |
| 10 | Neg/Pos | Neg | Neg |
| 11 | Pos | Neg | Pos |
| 12 | Pos | Pos | Pos |
| 13 | -- | Pos | Pos |
| 14 | -- | Pos | Pos |
| 15 | -- | Neg | Pos |
| 16 | -- | Neg | Pos |
| 17 | -- | Neg | Pos |
| 18 | -- | Neg | Pos |

(continued)

| Samples | BD GeneOhm | EuroClone RT-PCR | EuroClone New technology |
|---|---|---|---|
| 19 | -- | Neg | Pos |
| 20 | -- | Neg | Pos |
| 21 | -- | Neg | Pos |
| 22 | -- | Neg | Pos |
| 23 | -- | Pos | Pos |
| 24 | -- | Pos | Pos |
| 25 | Pos | Pos | Pos |
| 26 | Pos | Pos | Pos |
| 27 | Pos | Pos | Pos |
| 28 | Neg | inhibited | Pos |
| 29 | Pos | inhibited | Pos |
| 30 | Pos | Pos | Pos |
| 31 | Pos | Neg | Pos |
| 32 | Pos | Pos | Pos |
| 33 | Pos | Pos | Pos |
| 34 | Pos | inhibited | inhibited |
| 35 | Neg | Pos | Pos |
| 36 | Pos | Pos | Pos |
| 37 | Pos | Pos | Pos |
| 38 | Pos | Neg | Pos |
| 39 | Pos | Neg | Pos |
| 40 | Pos | inhibited | Pos |
| 41 | -- | -- | Pos |
| 42 | Pos | Pos | Pos |
| 43 | Pos | inhibited | Pos |
| 44 | Pos | Neg | Pos |
| 45 | Pos | Pos | Pos |
| 46 | Neg | Neg | Neg |
| 47 | Pos | Neg | Pos |
| 48 | Pos | inhibited | Pos |
| 49 | Pos | Pos | Pos |
| 50 | Pos | Neg | Pos |
| 51 | Pos | Pos | Pos |
| 52 | Pos | Neg | Pos |
| 53 | Pos | Neg | Pos |
| 54 | Pos | Neg | Pos |
| 55 | Pos | inhibited | Pos |
| 56 | Pos | Neg | Neg/Pos |
| 57 | Pos | Neg | Pos |
| 58 | Pos | Neg | Pos |
| 59 | Pos | Pos | Pos |
| 60 | Pos | Pos | Pos |
| 61 | Neg | Neg | Neg |
| 62 | Pos | Neg | Pos |
| 63 | Pos | Neg | Pos |
| 64 | Pos | Pos | Pos |
| 65 | Pos | Neg | Neg |
| 66 | Pos | Pos | Pos |
| 67 | Pos | Neg | Neg |
| 68 | Pos | Pos | Pos |

(continued)

| Samples | BD GeneOhm | EuroClone RT-PCR | EuroClone New technology |
|---|---|---|---|
| 69 | -- | Pos | Pos |
| 70 | Pos | Pos | Pos |
| 71 | Pos | Pos | Pos |
| 72 | Pos | Neg | Pos |
| 73 | Pos | Pos | Pos |
| 74 | Pos | Neg | Pos |
| 75 | Neg (Pos) | Neg | Neg |
| 76 | Pos | inhibited | Pos |
| 77 | Pos | Neg | Pos |
| 78 | Pos | Neg | Pos |
| 79 | Pos | Pos | Pos |
| 80 | Pos | Pos | Pos |
| 81 | Pos | Neg | Pos |
| 82 | Pos | Neg | Pos |
| 83 | Pos | Pos | Pos |
| 84 | Pos | Pos | Pos |
| 85 | Neg (Pos) | Neg | Neg |
| 86 | Pos | Pos | Pos |
| 87 | Neg | Pos | Pos |
| 88 | -- | inhibited | Neg |
| 89 | Pos | Pos | Pos |
| 90 | Pos | Pos | Pos |
| 91 | Neg | Neg | Pos |
| 92 | Neg | inhibited | Neg |
| 93 | Neg | Neg | Neg |
| 94 | -- | Pos | Pos |
| 95 | Pos | Neg | Pos |
| 96 | Pos | inhibited | Pos |

Data Analysis

Table 8 below summarizes the results listed in Table 7, for the kit based on the new technology and the kit based on the standard RT-PCR technology, respectively:

Table 8a

| Results of the new technology | | |
|---|---|---|
| | BD Positive | BD Negative |
| Positive.EC | 65 | 4 |
| Negative.EC | 2 | 4 |
| | | |
| Total number of samples | 96 | |
| Inhibited samples | 1 | |
| Non calculated samples | 20 | |

Table 8b

| Results of standard RT-PCR | | |
|---|---|---|
| | BD Positive | BD Negative |
| Positive.EC | 32 | 2 |

(continued)

|  | Results of standard RT-PCR | |
| --- | --- | --- |
|  | BD Positive | BD Negative |
| Negative.EC | 29 | 4 |
| Total number of samples | 96 | |
| Inhibited samples | 11 | |
| Non calculated samples | 18 | |

The total number of samples analyzed is 96. Among those, 15 samples were not analyzed with the reference method (BD Gene Ohm) and were not included in the following calculations, although they were considered further below in a comparison between EuroClone kits (standard RT-PCR vs. new technology). In addition to these 15 samples, 3 other samples giving discordant results with the reference method and 1 sample giving a discordant result with the kit of the new technology were not included in the calculations.

It can be immediately seen that the results discordant form the reference method (BD Gene Ohm) are 6 for the kit of the new technology and 31 for the kit based on the standard RT-PCR technology.

With regard to the kit of the new technology, the 6 discordant results include 2 samples that scored positive with the reference method and negative with the EuroClone test, while 4 samples scored negative with the reference method and positive with EuroClone test.

It is interesting to note that the reference method is able to detect the toxin B gene (tcdB gene) but not the toxin A gene, unlike EuroClone kits (both standard RT-PCR and the new technology) which are dual target and thus able to detect the presence of two target genes, and in particular tcdB tcdA. This may suggest that the 4 samples not detected by the standard method may belong to a tcdB-/tcdA+ strain; alternatively these samples might contain very low amounts of target DNA. In particular, the first possibility (tcdB-/tcdA+ strain) appears likely for 2 out of 4 samples that were recognized positive also by the EuroClone kit based on the standard RT-PCR technology (n° 87 and 35): such kit showed a lower diagnostic performance on these clinical samples, in terms of sensitivity, and it is likely that the failure of the reference kit to detect these targets has to be attributed to unique characteristics of the strain used. The 2 other samples turned out to be negative and inhibited, respectively (n° 91 and 28) with the RT-PCR EuroClone kit; in this case detection with the reference kit may have actually failed because of the greater sensitivity of the kit of the new technology.

However, the most evident data from the above results concerns the difference in sensitivity between the EuroClone kit based on standard RT-PCR and the one based on the new technology: as mentioned above, the standard RT-PCR kit failed to detect 29 samples that scored positive with the reference test. Twentyseven out of these 29 samples were subsequently confirmed as positives using the EuroClone kit based on the new technology.

NEW TECHNOLOGY:

DIAGNOSTIC SENSITIVITY: 97.01%

A further element that demonstrates a higher performance of the new technology is the lower number of inhibited samples: 11 inhibited samples were obtained using the standard RT-PCR technology , whereas only 1 inhibited sample was obtained using the kit based on the new technology. Considering the complexity of the starting biological matrix (faecal samples), this result however indicates that transition to the new technology allows greater robustness of the system. In particular it is plausible that the first phase where only primers work, under highly specific conditions, to enrich the target DNA sample can reduce the inhibitory action of interfering substances on the probe+primer system that becomes active only in the subsequent step of the amplification protocol.

Finally, it should be considered the inherent variability of the DNA extraction processes that may however affect performance: in other words, it is plausible that the high number of inhibited samples obtained with standard RT-PCR is due not only to poor performance of the kit but also to problems in the course of extraction.

Another significant result on increased performance of the new technology in terms of sensitivity comes from the analysis of data from 15 samples comparing the EuroClone RT-PCR kit and the EuroClone kit based on the new technology, while no data with the reference method was available (BD Gene Ohm) for these samples. Also in this case there is a clear difference in term sensitivity: 8 samples that scored negative by the standard RT-PCR kit tested actually positive using the kit based on the new technology.

**ANALYTICAL PERFORMANCE**

ANALYTICAL SENSITIVITY

In this test, a genomic sample of C. difficile, quantified at 10e4 copies/uL (ABI Advanced Biotechnologies Inc.), was tested at different dilutions in order to determine test sensitivity:

Table 9:

|  | CT | |
|---|---|---|
|  | **Specific Probe** | **C.I.** |
| Samples |  |  |
| 100 copies | 18.28 | 20.43 |
| 10 copies | 21.24 | 20.30 |
| 1 copy | 24.75 | 21.15 |
| 0,1 copies | -- | 20.96 |
| 0,01 copies | -- | 20.14 |
| 0,001 copies | -- | 22.63 |
| 0,0001 copies | -- | 19.27 |
| CP | 14.52 | 23.89 |
| Neg | -- | 21.17 |

As mentioned above, the analytical sensitivity of this new technology, calculated from genomic samples (hence not real clinical samples, and devoid of any possible interference) is comparable to a standard RT-PCR. Thus the method according to the invention is able to improve performance in terms of sensitivity on real clinical samples, where the first step of sample pre-enrichment actually allows RT-PCR to work more efficiently in the subsequent step, making the system more robust and improving performance in diagnostic sensitivity.

ANALYTICAL SPECIFICITY

Table 2 summarizes the results obtained from samples that were negative for CD and positive for other pathogens, in order to verify the specificity of the kit based on the new technology:

Table 10:

|  | CT | |
|---|---|---|
|  | **Specific Probe** | **C.I.** |
| Campioni |  |  |
| Asperg. | -- | 21.73 |
| Salmonella | -- | 21.24 |
| Listeria | -- | 21.49 |
| E.Coli | -- | 21.09 |
| Legionella | -- | 20.63 |
| Adenovirus | -- | 21.33 |
| ChlamydiaT. | -- | 18.39 |
| Campilob.J. | -- | 20.86 |
| C.P. | 14,16 | 20.69 |
| Neg | -- | 20.33 |

INTER-ASSAY REPRODUCIBILITY

To assess inter-assay reproducibility, analyses were carried on samples and controls as listed in Table 11:

Table 11

| samples | I ASSAY CT | | II ASSAY CT | | III ASSAY CT | | IV ASSAY CT | | Results | |
|---|---|---|---|---|---|---|---|---|---|---|
| | Specific probe | C.I. | Specific probe | C.I. | Specific probe | C.I. | Specific probe | C.I. | Specific probe | C.I. |
| 70 | 17.67 | 23.46 | 18.07 | 20.50 | 17.88 | 20.13 | 18.39 | 20.61 | **18,00 +/-0.264 c.v. 1.4%** | **21,17 +/-1.33 c.v. 6.28%** |
| 71 | 21.76 | 22.22 | 21.27 | 20.02 | 20.81 | 21.19 | 21.46 | 21.03 | **21,325 +/-0.344 c.v. 1.6%** | **21.11 +/- 0.779 c.v. 3.69%** |
| 46 | -- | 20.33 | -- | 20.41 | -- | 19.66 | -- | 21.40 | **--** | **20.45 +/- 0.621 c.v. 3.03%** |
| 93 | -- | 20.46 | -- | 22.35 | -- | 19.26 | -- | 19.93 | **--** | **20.48 +/- 0.924 c.v. 4.5%** |
| CP | 13.99 | 22.50 | 14.32 | 20.44 | 14.33 | 19.26 | 14.18 | 18.75 | **14.21 20.24 +/-0.137 0.968%** | **c.v. 20.24 +/-1.44 c.v. 7.1%** |
| Neg | -- | 19.79 | -- | 21.00 | -- | 19.82 | -- | 21.04 | **--** | **20.42 +/- 0.607 c.v. 2.97%** |
| Positive Samples +<br>Negative Samples -<br>Controls | | | | | | | | | | |

INTRA-ASSAY REPRODUCIBILITY

To assess the intra-assay reproducibility samples and controls were tested in quadruplicate as shown in Table 12:

| CT | | | | | | | | |
|---|---|---|---|---|---|---|---|---|
| | FAM | FAM | FAM | FAM | HEX | HEX | HEX | HEX |
| Samples | | | | | | | | |
| 70 | 18.60 | 17.46 | 17.13 | 17.34 | 18.75 | 20.80 | 20.31 | 20.25 |
| 71 | 20.38 | 21.58 | 22.85 | 20.92 | 20.30 | 21.49 | 20.13 | 20.35 |
| 46 | -- | -- | -- | -- | 20.12 | 20.50 | 19.81 | 21.40 |
| 93 | -- | -- | -- | -- | 20.35 | 22.19 | 20.56 | 21.05 |
| **CP** | 14.21 | 13.93 | 14.27 | 14.30 | 20.34 | 23.58 | 21.54 | 21.56 |
| **Neg** | -- | -- | -- | -- | 20.91 | 21.03 | 20.41 | 21.77 |
| Positive Samples<br>Negative Samples<br>Controls | | | | | | | | |

Sample 70: 17.63+/-0.571 CV 3.2 % 20.03+/-0.767 CV 3,8 %
Sample 71: 21.43+/-0.922 CV 4.3 % 20.56+/-0.539 CV 2.6 %
Sample 46: 20.45+/-0.596 CV 2.9 %
Sample 93: 21.03+/-0.712 CV 3.3 %
C. Positive : 14.18+/-0.146 CV 1.0 % 21.75+/-1.163 CV 5.3 %
C. Negative : 21.03+/-0.486 CV 2.3 %

## EXAMPLE 4

## COMPARISON OF STANDARD RT-PCR WITH THE NEW TECHNOLOGY

ADVANCED technology was applied for detection of DNA of M. Genitalium in clinical samples from two different biological matrices: urine and cervical swabs. Such samples were analyzed using a kit based on standard RT-PCR technology (developed by EuroClone) and with a kit based on the new technology. Results were compared with a conventional reference system (in-house kit for detection of M. genitalium directed against the MgPa gene see Jensen et al. 2004)

### Materials and methods

Samples analyzed with the Euroclone kit (classical RT-PCR and new technology) were extracted with the DuplicaPrep automated DNA extraction system according to instructions described in the instruction manual of the instrument.

For realization of the test, primers and probes were selected in order to perform a first step a) of amplification, where only primers work (high-annealing temperature), and a second step b) where also the Probe works (low annealing temperature).

The following oligonucleotide sequences were selected for two target genes of M. genitalium:

**M.genitalium Pa gene (strain M2288)**

M.G.Fwd:5'-GAGAAATACCTTGATGGTCAGCA          55
M.G.Rev:5'- ATATCATATAAAGCTCTACCGTTGTTATC     54.1

(continued)

Probe:5'-FAM-ACTTTGCAATCAGAAGGT-BHQ1          47.06

**(Mycoplasma genitalium strain M2288 16S ribosomal RNA gene, partial**

M.G.16s Fwd:5'-GTAATACATAGGTCGCAAGCGTTATC          57.5
M.G.16s Rev:5'- CACCACCTGTCACTCGGTTAA          57.6
M.G.16s Probe2:5'-FAM-TAGATACTAGCTGTCGGAG-BHQ1          44.3
(Nested)

Internal Control

MPL515 Fwd:5'-ACAGAGCGAACCAAGAATG          52.9
MPL515 Rev: 5'-TAGCCTGGATCTCCTTGGT          53.5
MPL Mut2:5'-HEX-AGGAAACTGCTTCCTCAGCAG-BHQ1          58

The reaction mixture is as shown in Table 13 below:

| Mycoplasma Genit. | | | | | | Sample s | Run |
|---|---|---|---|---|---|---|---|
| Primer mix | | | | | | | 10 |
| Initial conc. | | Reagent | Final conc. | | Lot | Vol (ul) | 1 | |
| 100 uM | | FWD 16s | 0.2 uM | | | 0.05 | 0.05 | 0.5 |
| 100 uM | | REV 16s | 0.2 uM | | | 0.05 | 0.05 | 0.5 |
| 100 uM | | Probe 16s | 0.2 uM | | | 0.05 | 0.05 | 0.5 |
| 100 uM | | FWD MaPa | 0.1 uM | | | 0.025 | 0.025 | 0.25 |
| 100 uM | | REV MaPa | 0.1 uM | | | 0.025 | 0.025 | 0.25 |
| 100 uM | | Probe MaPa | 0.1 uM | | | 0.025 | 0.025 | 0.25 |
| 100 uM | | FWD C.I. | 0.2 uM | | | 0.05 | 0.05 | 0.5 |
| 100 uM | | REV C.I. | 0.2 uM | | | 0.05 | 0.05 | 0.5 |
| 100 uM | | Probe C.I. | 0.2 uM | | | 0.05 | 0.05 | 0.5 |
| 10 % | | Glycerol | 1.2 % | | | 3 | 3 | 30 |
| | | H2O | as needed | | | 6.661 | 7 | 66.61 |
| | | | | | Final volume | 10 | 10 | 100 |

| Reaction master mix | | | | Samples |
|---|---|---|---|---|
| Reagent | Final conc. | Lot | Vol (ul) | 10 |
| 2x Master Mix probes | 1:1 | smar taq 0,2U/ul | 10 | 100 |
| 2x Primer mix | 1:1 | see above | 10 | 100 |
| | Final total volume | | 20 | 200 |
| | Internal control | | 1 | |
| | Template DNA | | 5 | |

The samples prepared as described above are applied to the SmartCycler (Cepheid) real-time instrument that is programmed to execute the following amplification protocol:

**Thermal profile: 95°C for 5 min.**
**(95 °C for 15 sec., 62 °C for 1 min.)x 10 cycles**
**(95°C for 15 sec., 52°C for 1 min.)x 45 cycles**

Results

LEGEND

| | |
|---|---|
| | EC POS/BD POS |
| | EC POS/BD NEG |
| | EC NEG/BD POS |
| | EC NEG/BD NEG |
| | NOT CALCULATED |
| | INHIBITED |
| *** | lack of material |

Results are shown below in Table 14:

| SAMPLE | Reference test (in house RT-PCR kit) (Ct) | EuroClone RT-PCR standard (Ct) | EuroClone New technology (Ct) | Biological matrix |
|---|---|---|---|---|
| 1 (69470) | 28.09 | 29 | 19 | Cervix-urine |
| 2 (66626) | 28.09 | 29 | 19 | Urine |
| 3 (65877) | 40.61 | 39 | 29 | Urine |
| 4 (69444) | 38.08 | 35 | 25 | Urine |
| 5 (70659) | 27.31 | 26 | 15 | Urethra-cervix |
| 6 (68580) | 40.51/neg/neg | Negative | 32/neg | Cervix-urine |
| 7 (67000) | 32.48 | 32 | 22 | Cervix-urine |
| 8 (70093) | 37.08 | Negative | 26 | Urine+swab*** |
| 9 (68583) | 40.94 | Negative | 30 | Urine |
| 10 (66563) | 39.91 | Negative | Negative | |
| 11 (MG+) | 33.97 | Negative | 25 | Sample contr. |
| 12 (70088) | 36.88 | Negative | 27 | Urine |
| 13 (70518) | 29.27 | 29 | 17 | Cervix-urine |

| SAMPLE | Reference test (in house RT-PCR kit) (Ct) | EuroClone RT-PCR standard (Ct) | EuroClone New technology (Ct) | Biological matrix |
|---|---|---|---|---|
| 14 (68578) | 28.17 | 30 | 20 | Urine |
| 15 (69473) | 33.64 | 34 | 24 | Urine |
| 16 (70521) | 29.11 | 29 | 18 | Urine |
| 17 (67634) | 40.63 | 38 | 29 | Cervix-urine |
| 18 (69427) | 34.45 | 34 | 24 | Urine |
| 19 (68104) | 20.06 | 28 | 18 | Urine |
| 20 (68565) | 36.25 | 40 | 29 | Urine |
| 21 (67632) | 37.82 | Negative | Negative | Cervix |
| 22 (68106) | 36.88 | 39 | 28 | Urine |
| 23 (68099) | 33.7 | 38 | 26 | Urine |
| 24 (68579) | 36.92 | 35 | 24 | Urine+swab |
| 25 (69428) | 31.44 | 32 | 21 | Cervix-urine |
| 26 (70085) | 31.91 | 35 | 30 | Urine |
| 27 (Equalis 1) | 33.96 | 34 | 23 | Urine |
| 28 (Equalis 2) | Negative | Negative | Negative | Urine |
| 29 (Equalis 3) | Negative | Negative | Negative | Urine |
| 30 (Equalis 4) | 39.71 | Negative | Negative | Urine |
| 31 (Equalis 5) | 30.54 | 30 | 20 | Urine |
| 32 (Equalis 6) | 36.93 | 39 | 29 | Urine |
| 33 (67641) | 39.94 | 40 | 28 | Cervix-urine |
| 34 (68095) | 32.27 | 32 | 22 | Cervix-urine |
| 35 (66516) | 29 | Negative | 22 | Urethra *** |

Data Analysis

Table 15 below summarizes the results listed in Table 14, for the kit based on the new technology and the kit based on the standard RT-PCR technology, respectively:

Table 15a

| Results of the new technology | | |
|---|---|---|
| | Positive Reference | Negative Reference |
| Positive EC | 29 | 0 |
| Negative EC | 3 | 2 |
| | | |
| Total number of samples | 35 | |
| Inhibited samples | 0 | |
| Non calculated samples | 1 | |

Table 15b

| Results of standard RT-PCR | | |
|---|---|---|
| | Positive Reference | Negative Reference |
| Positive EC | 24 | 0 |
| Negative EC | 8 | 2 |
| | | |
| Total number of samples | 35 | |
| Inhibited samples | 0 | |
| Non calculated samples | 1 | |

The total number of samples analyzed is 35. Among these, 1 sample was not included in the following calculations because produced conflicting results with the reference method (low positive and negative in 2 tests repeated later).

It can be immediately seen that the discordant results form the reference method (in-house kit directed against the MgPa gene, see Jensen et al. 2004) are 3 for the kit of the new technology and 8 for the kit based on the standard RT-PCR technology.

As for the kit of the new technology, 3 discordant samples were found that tested positive with the reference method and negative with the EuroClone test.

It should be emphasized that 2 of these discordant samples (No. 67632 and 66563) are characterized by a small amount of starting biological material. The extraction step by DuplicaPrep prior to analysis by EuroClone further reduced the amount of available material, evidently resulting in an amount of target DNA that was below the detection limit. The third discrepant sample is a low positive (Ct 39.71), although it is important to note that the kit of the new technology was able to detect the presence of target DNA in all other low positive clinical samples that were tested.

Therefore the most clear result from the above data is the increase of diagnostic sensitivity obtained with the new technology. In fact, we identified 8 positive clinical samples with the reference method and negative samples with the kit based on the standard RT-PCR technology. Five of these 8 samples were correctly identified as positives using the kit of the new technology, while two samples had too little starting material (see above) and sample No. 39 was low positive (see above).

**ANALYTICAL PERFORMANCE**

ANALYTICAL SENSITIVITY

In this test, a genomic sample of M. genitalium, quantified at 10e4 copies/uL (ABI Advanced Biotechnologies Inc.), was tested at different dilutions in order to determine test sensitivity.

Table 16

| Quantified Genomic | FAM CT | HEX CT |
|---|---|---|
| 100000 copies | 13.65 | -- |
| 10000 copies | 16.90 | 22.49 |
| 1000 copies | 20.70 | 23.49 |

(continued)

| Quantified Genomic | FAM CT | HEX CT |
| --- | --- | --- |
| 100 copies | 23.54 | 23.29 |
| 10 copies | 27.04 | 23.60 |
| 5 copies | 27.12 | 23.19 |
| 2,5 copies | 31.26 | 23.23 |
| 1 copy | 33.78 | 23.28 |
| 0,5 copies | -- | 23.28 |
| 0,1 copies | -- | 23.83 |
| blank | -- | 23.72 |

As with the above examples, the analytical sensitivity of this new technology, calculated from genomic samples (hence not real clinical samples, and devoid of any possible interference) is comparable to a standard RT-PCR. Thus the method according to the invention is able to improve performance in terms of sensitivity on real clinical samples, where the first step of sample pre-enrichment actually allows RT-PCR to work more efficiently in the subsequent step, making the system more robust and improving performance in diagnostic sensitivity.

ANALYTICAL SPECIFICITY

Table 17 summarizes the results obtained from samples that were negative for MG and positive for other pathogens, in order to verify the specificity of the kit:

Table 17:

| Samples | FAM CT | HEX CT |
| --- | --- | --- |
| Mycoplasma genitalium (1000 copies) | 20.84 | 22.93 |
| Mycoplasma genitalium (100 copies) | 23.50 | 23.08 |
| Mycoplasma genitalium (10 copies) | 30.81 | -- |
| Tricomonas Vaqinalis | -- | 23.81 |
| Chlamidia Trachomatis | -- | 22.36 |
| Ureaplasma Urealiticum | -- | 22.56 |
| Clostridium Difficile | -- | 23.22 |
| Neisseria Gonorrea | -- | 22.87 |
| Candida Albicans | -- | 22.56 |
| Mycoplasma Hominis | -- | 22.98 |
| C+ (Mycoplasma genitalium:100 copies) | 24.20 | 23.05 |
| blank | -- | 22.95 |

INTER-ASSAY REPRODUCIBILITY

To assess inter-assay reproducibility, analyses were carried on samples and controls in different assays, as listed below in Table 18:

| SAMPLES | FAM CT 1 | FAM CT 2 | FAM CT 3 | FAM CT 4 | HEX CT 1 | HEX CT 2 | HEX CT 3 | HEX CT 4 | FAM CT | HEX CT |
|---|---|---|---|---|---|---|---|---|---|---|
| **1000 copies** | 20.47 | 20.31 | 20.33 | 20.23 | 23.35 | 23.28 | 23.08 | 23.30 | 20.335 +/- 0.099833 | 23.2525 +/- 0.118708 |
| **100copies** | 23.73 | 23.88 | 23.61 | 23.79 | 22.56 | 22.38 | 22.23 | 22.83 | 23.7525 +/- 0.113248 | 22.5 +/- 0.25807 |
| **NEG** | -- | -- | -- | -- | 22.69 | 22.85 | 22.67 | 23.06 | -- | 22.8175 +/- 0.180624 |
| **NEG** | -- | -- | -- | -- | 22.75 | 23.47 | 23.04 | 23.34 | -- | 23.15+/- 0.321766 |
| **CP** | 23.81 | 23.46 | 23.68 | 23.28 | 22.59 | 22.90 | 22.62 | 22.49 | 23.5575 +/- 0.234716 | 22.65 +/- 0.175689 |
| **Neg. control** | -- | -- | -- | -- | 23.14 | 23.11 | 23.32 | 23.09 | -- | 23.165+/- 0.105357 |

INTRA-ASSAY REPRODUCIBILITY

To assess the intra-assay reproducibility samples and controls were tested in quadruplicate as shown below in Table 19:

Table19:

| SAMPLES | FAM CT 1 | FAM CT 2 | FAM CT 3 | FAM CT 4 | HEX CT 1 | HEX CT 2 | HEX CT 3 | HEX CT 4 | FAM CT | HEX CT |
|---|---|---|---|---|---|---|---|---|---|---|
| **1000 copies** | 20.47 | 20.66 | 20.37 | 20.19 | 23.35 | 23.11 | 22.46 | 25.99 | 20.4225 +/- 0.196193 | 23.7275 +/-1.554485 |
| **100copies** | 23.73 | 22.91 | 22.71 | 23.22 | 22.56 | 22.95 | 23 | 22.92 | 23.1425+/- 0.444325 | 22.8575 +/-0.20106 |
| **NEG** | -- | -- | -- | -- | 22.69 | 23.20 | 23.07 | 22.84 | -- | 22.95 +/-0.228473 |
| **NEG** | -- | -- | -- | -- | 22.75 | 23.31 | 23.19 | 23.15 | -- | 23.1 +/-0.243036 |
| **CP** | 23.81 | 24.10 | 23.36 | 24.17 | 22.59 | 23.19 | 23.82 | 22.88 | 23,86 +/-0,367967 | 23.12+/-0.527067 |
| **Neg. control** | -- | -- | -- | -- | 23.14 | 22.70 | 23.19 | 23.08 | -- | 23.0275-+/- 0.222916 |

## EXAMPLE 5

The method according to the present invention was also applied for detection of DNA of Hepatitis C virus, Hepatitis B virus, Toxoplasma gondii, Hepes simplex virus, Varicella zoster, Epstein-Barr virus, Citomegalovirus, Enterovirus, BK virus, JC virus.

The clinical samples were taken from different biological matrices: such as urine, stool and cervical swabs. Such samples were analyzed using a kit based on standard RT-PCR technology (developed by EuroClone) and with a kit based on the new technology. Results were compared with a conventional reference systems.

The following primer and probe sequences were used:

### Hepatitis C Virus

HCV Fwd:5'-CCTCCCGGGAGAGCCATAGT (SEQ ID N°7)
HCV Rev:5'-ATGRTGCACGGTCTACGAGACCT (SEQ ID N°8)
HCV-Probe2 :5'-Fam-TGTACTCACCGGTTCC-BHQ1 (SEQ ID N°9)

### Hepatitis B Virus

HBV Fwd:5'-TCACTCACCAACCTSYTGTCCTCC (SEQ ID N°10)
HBV Rev :5'-AGGACAMACGGGCAACATACCT (SEQ ID N°11)
HBV Probe:5'-FAM-TATCGYTGGATGTGTCTG-BHQ1 (SEQ ID N°12)

### TOXOPLASMA GONDII

TOXO Fwd B: 5'-TGTGCTTGGAGCCACAGAAGG (SEQ ID N°13)
TOXO Rev B: 5'- TCGTCTRGATCGCATTCCGGTG (SEQ ID N°14)
TOXO probeB: 5'-Fam- AGAGATATCAGGACTGTAGATG -BHQ1 (SEQ ID N°15)

### HERPES SIMPLEX VIRUS 1

HSV1-2 FWD:5'-TGTTCTCGTTCCTYACWGCCTCC (SEQ ID N°16)
HSV1-2 REV:5'-ACGTAACGCACGCTMGGGTGTG (SEQ ID N°17)
PROBE HSV-1:5'-FAM- TCCACACCTTATCGTTT -BHQ1 (SEQ ID N°18)

### HERPES SIMPLEX VIRUS 2

HSV2 FWD:5'-GATATCCTCTTTATCATCAGCACCACCA (SEQ ID N°19)
HSV2 REV:5'-GTAACGCACGCTMGGGTGC (SEQ ID N°20)
HSV-2PROBE: 5'-FAM-ACCAGACAAACGAACG-BHQ1 (SEQ ID N°21)

### VARICELLA-ZOOSTER VIRUS

VZV FWD: 5'-GAAGTTCCCCCCGTTCGC (SEQ ID N°22)
VZV REV: 5'-CCTGGACTTGAAGATGAACTTAATGAAGC (SEQ ID N°23)
VZV PROBE: 5'-FAM- ACAACTGCAGTATATATCGTC -BHQ1 (SEQ ID N°24)

### EPSTEIN-BARR VIRUS

EBV FWD: 5'-AAACCTCAGGACCTACGCTGC (SEQ ID N°25)
EBV REV : 5'-CACAGACACCGTCCTCACCAC (SEQ ID N°26)
EBV-Probe: 5'-FAM- TAGAGGTTTTGCTAGGGA-BHQ1 (SEQ ID N°27)

### CITOMEGALOVIRUS

CMV UL123 Fwd: 5'-AGCGCCGCATTGAGGA (SEQ ID N°28)
CMV UL123 Rev: 5' -CAGACTCTCAGAGGATCGGCC (SEQ ID N°29)
CMV UL123 Probe3:5'-Fam-ATCTGCATGAAGGTCTTT-BHQ1 (SEQ ID N°30)

**HUMAN HERPES VIRUS 6**

HHV6 FWD: 5'-TGTTAGGRTATACCGATGTGCGTGATC (SEQ ID N°31)
HHV6 REV: 5'-CACGTAAGCTTGCACAATGCCA (SEQ ID N°32)
HHV6 PROBE FAM-TTCCRAATCTATTGCCTC-BHQ1 (SEQ ID N°33)

**HUMAN HERPES VIRUS 7**

HHV7 FWD:5'-CGGCGTTTTACTCGGAACTCCT (SEQ ID N°34)
HHV7 REV:5'-TCCCCATAACAAATGTGCCATAAGA (SEQ ID N°35)
HHV7 PROBE: 5'-FAM-TACGATTCAGATTTTGTCC-BHQ1 (SEQ ID N°36)

**HUMAN HERPES VIRUS 8**

HHV8 FWD: 5'- AGCCGAAAGGATTCCACCATTG (SEQ ID N°37)
HHV8 REV: 5'-GGTGGTATATAGATCAAGTTCCGCCA (SEQ ID N°38)
HHV8 Probe: 5'-FAM-ACATCTACTCCAAAATATCG-BHQ1 (SEQ ID N°39)

**ENTEROVIRUS**

ENT.FWD:5'-GCCCCTGAATGCGGCTAAT (SEQ ID N°40)
ENT.REV:5'-CACCGGATGGCCAATCCAA (SEQ ID N°41)
ENT. Probe: 5'FAM-ACCCAAAGTAGTCGGTT-BHQ1 (SEQ ID N°42)

**BK Virus**

BKV Fwd: 5'-AATGCAGTAGCAATCTATCCAAACCAA (SEQ ID N°43)
BKV Rev: 5'-GCAAGGAWTGGCCTATTTGTTCCAAA (SEQ ID N°44)
BKV Probe: 5'-Fam-ATGCCTTAATCTAAGCTGA-BHQ1 (SEQ ID N°45)

**JC Virus**

JCV Fwd: 5'-AGAGTGYTGGGATCCTGTGTTTT (SEQ ID N°46)
JCV Rev: 5'-ATGARAAGTGGGATGAAGACCTGTTT (SEQ ID N°47)
JCV Probe: 5'-Fam-TGGCAAACATTTCTTCAT-BHQ1 (SEQ ID N°48)

**CHLAMYDIA TRACHOMATIS**

C.T. FWD 16s.: 5'-TGGCGATATTTGGGCATCCGAGTAAC (SEQ ID N°49)
C.T. REV 16s.: 5'- TACGCTCAAATCCAGCGGGTATTAACCG (SEQ ID N°50)
C.T. Probe 16s.:5'- FAM -ACCGAAAGGTCCTAAGA-BHQ1 (SEQ ID N°51)
CT P.C.Fwd:5'-AGGATCACTTCTCCTCAACAACTTTTTCAT (SEQ ID N°52)
CT P.C.Rev.:5'-TTTGTAGAGTTTGGTTGGGGAGGTTTA (SEQ ID N°53)
CT P.C.Probe:5'-Fam-TTCTGCTTACAATGCTCTT-BHQ1 (SEQ ID N°54)

**CLOSTRIDIUM DIFFICILE**

C.D.TA Fwd:5'-CAATGGGCTGATATTAATGCAGAAT (SEQ ID N°55)
C.D.TA Rev: 5'-TCATCTATTGTARGTACTGTAGGTTTATTG (SEQ ID N°56)
C.D.TA Probe: 5'-Fam-ACAGCTACTAGAGGAAGAGAT-BHQ1 (SEQ ID N°57)
C.D.TB Fwd1:5'-TGAGGACATATCAGAGACTGATGAGG (SEQ ID N°58)
C.D.TB Rev1: 5'-TAGCATATTCAGAGAATATTGYYTTTTCAG (SEQ ID N°59)
C.D.TB Probe1: 5'-Fam-CTGGAGAATCTATATTTGTAGAA-BHQ1 (SEQ ID N°60)

**MYCOPLASMA GENITALIUM**

M.G.Fwd:5'-GAGAAATACCTTGATGGTCAGCA (SEQ ID N°61)
M.G.Rev:5'- ATATCATATAAAGCTCTACCGTTGTTATC (SEQ ID N°62)

Probe:5'-FAM-ACTTTGCAATCAGAAGGT-BHQ1 (SEQ ID N°63)
M.G.16s Fwd:5':-GTAATACATAGGTCGCAAGCGTTATC (SEQ ID N°64)
M.G.16s Rev:5'- CACCACCTGTCACTCGGTTAA (SEQ ID N°65)
M.G.16s Probe2:5'-FAM-TAGATACTAGCTGTCGGAG-BHQ1 (SEQ ID N°66)

## Claims

1. A real time PCR-based method for detecting a target nucleic acid sequence in a sample consisting essentially of:

a) a pre amplification step comprising the amplification of the target nucleic acid sequence in the presence of at least two amplification primers and a probe, wherein the amplification primers have a melting temperature (Tm) of at least 5°C higher than the melting temperature (Tm) of the probe, and
wherein such pre-amplification step is carried out at a temperature higher than the melting temperature (Tm) of the probe such that the primers hybridize to the target nucleic acid sequence and the probe substantially does not hybridize,
b) a simultaneous amplification and detection step comprising a real time PCR step which is carried out at a temperature lower than the temperature of step a) allowing simultaneous hybridization of the primers with amplification of the target nucleic acid sequence and hybridization of the probe with detection of the target nucleic acid sequence.

2. Method according to claim 1, wherein said melting temperature of the amplification primers is 10°C higher than said probe melting temperature.

3. Method according to claim 1, wherein said melting temperature of the amplification primers is 20°C higher than said probe melting temperature.

4. Method according to anyone of claims 1-3, wherein said melting temperature of the primers is from 54 to 70°C and said probe melting temperature is from 42 to 48°C.

5. Method according to anyone of claims 1-3, wherein said melting temperature of the primers is from 64 to 70°C and said probe melting temperature is from 42 to 48°C.

6. Method according to anyone of claims 1 to 5, **characterized in that** said pre-amplification step a) comprises:

   providing an amplification reaction mixture comprising a target nucleic acid sequence, at least two primers, a probe, a polymerization inducing agent, deoxyribonucleoside triphosphates (dNTP); subjecting said amplification reaction mixture to a plurality of thermal cycles, comprising

   - a denaturation step for separating the two strands of the target nucleic acid to obtain two templates for the synthesis of a complementary nucleic acid,
   - a hybridization step for annealing each primers with the correspondent target nucleic acid
   - an extension step of the complementary strand from the primer by the polymerization inducing agent.

7. Method according to anyone of claims 1 to 6 **characterized by** the fact that said real time-PCR step b) of the target nucleic acid sequence comprises

   - treating the amplification mixture containing the hybridized probe under conditions for amplifying the target nucleic acid sequence,
   - determining the amount of said signal produced in the preceding step and
   - determining if amplification has occurred.

8. Method according to anyone of claims 1 to 7 **characterized by** the fact that said target nucleic acid sequence is a DNA sequence of a microorganism, preferably a pathogen microorganism.

9. Method according to claim 8 **characterized in that** said microorganism is selected from the group consisting of Chlamydia Trachomatis, Hepatitis C virus, Hepatitis B virus, Toxoplasma gondii, Hepes simplex virus, Varicella zoster, Epstein-Barr virus, Citomegalovirus, Enterovirus, BK virus, JC virus, Chlamydia phage, Clostridium difficile, and Mycoplasma genitalium.

10. Method according to claim 8 **characterized in that** said microorganism is selected from the group consisting of Chlamydia Trachomatis, Clostridium difficile, and Mycoplasma genitalium.

11. Use of a kit for detecting a target nucleic acid sequence in a sample in the method according to anyone of Claims 1-10, wherein said kit comprises at least two primers and a probe, and wherein the at least two primers have a melting temperature (Tm) of at least 5°C higher than the melting temperature (Tm) of the probe.

12. Use of a kit according to Claim 11 said kit further comprising deoxyribonucleoside triphosphates (dNTPs), a polymerization inducing agent, and an instruction leaflet.

13. Use of a kit according to anyone of claims 11 or 12 said kit further comprising salts and buffers.

14. Use of a kit according to anyone of claims 11 to 13 said kit further comprising a target nucleic acid sequence.

**Patentansprüche**

1. Echtzeit-PCR-basiertes Verfahren zum Detektieren einer Ziel-Nukleinsäuresequenz in einer Probe, im Wesentlichen bestehend aus:

   a) einem Prä-Amplifikationsschritt, umfassend die Amplifikation der Ziel-Nukleinsäuresequenz in Gegenwart von mindestens zwei Amplifikations-Primern und einer Sonde, wobei die Amplifikations-Primer eine Schmelztemperatur (Tm) von mindestens 5°C höher als die Schmelztemperatur (Tm) der Sonde aufweisen, und wobei solch ein Prä-Amplifikationsschritt bei einer Temperatur höher als die Schmelztemperatur (Tm) der Sonde durchgeführt wird, so dass die Primer an die Ziel-Nukleinsäuresequenz hybridisieren und die Sonde im Wesentlichen nicht hybridisiert,
   b) einem simultanen Amplifikations- und Detektionsschritt, umfassend einen Echtzeit-PCR-Schritt, der bei einer Temperatur niedriger als die Temperatur von Schritt a) durchgeführt wird, wobei simultanes Hybridisieren der Primer mit Amplifikation der Ziel-Nukleinsäuresequenz und Hybridisierung der Sonde mit Detektion der Ziel-Nukleinsäuresequenz zugelassen wird.

2. Verfahren nach Anspruch 1, wobei die Schmelztemperatur der Amplifikations-Primer 10°C höher als die Schmelztemperatur der Sonde ist.

3. Verfahren nach Anspruch 1, wobei die Schmelztemperatur der Amplifikations-Primer 20°C höher als die Schmelztemperatur der Sonde ist.

4. Verfahren nach einem der Ansprüche 1-3, wobei die Schmelztemperatur der Primer von 54 bis 70°C ist und die Schmelztemperatur der Sonde von 42 bis 48°C ist.

5. Verfahren nach einem der Ansprüche 1-3, wobei die Schmelztemperatur der Primer von 64 bis 70°C ist und die Schmelztemperatur der Sonde von 42 bis 48°C ist.

6. Verfahren nach einem der Ansprüche 1 bis 5, **dadurch gekennzeichnet, dass** der Prä-Amplifikationsschritt a):

   Bereitstellen einer Amplifikations-Reaktionsmischung, umfassend eine Ziel-Nukleinsäuresequenz, mindestens zwei Primer, eine Sonde, ein Polymerisations-induzierendes Mittel, Desoxyribonukleosidtriphosphate (dNTP); Unterziehen der Amplifikations-Reaktionsmischung einer Vielzahl an Temperaturzyklen, umfassend

      - einen Denaturierungsschritt zum Trennen der zwei Stränge der Ziel-Nukleinsäure, um zwei Template zur Synthese einer komplementären Nukleinsäure zu erhalten,
      - einen Hybridisierungsschritt zum Annealing jedes Primers mit der entsprechenden Ziel-Nukleinsäure,
      - einen Extensionsschritt des komplementären Strangs von dem Primer durch das Polymerisations-induzierende Mittel,

   umfasst.

7. Verfahren nach einem der Ansprüche 1 bis 6, **gekennzeichnet durch** die Tatsache, dass der Echtzeit-PCR-Schritt b) der Ziel-Nukleinsäuresequenz

      - Behandeln der Amplifikationsmischung, die die hybridisierte Sonde enthält, unter Bedingungen zum Amplifizieren der Ziel-Nukleinsäuresequenz,
      - Bestimmen der Höhe des Signals, das **durch** den vorangehenden Schritt erzeugt wurde und
      - Bestimmen ob Amplifikation stattgefunden hat, umfasst.

8. Verfahren nach einem der Ansprüche 1 bis 7, **gekennzeichnet durch** die Tatsache, dass die Ziel-Nukleinsäuresequenz eine DNA-Sequenz eines Mikroorganismus, bevorzugt eines Pathogen-Mikroorganismus, ist.

9. Verfahren nach Anspruch 8, **dadurch gekennzeichnet, dass** der Mikroorganismus ausgewählt ist aus der Gruppe bestehend aus Chlamydia trachomatis, Hepatitis-C-Virus, Hepatitis-B-Virus, Toxoplasma gondii, Herpes-simplex-Virus, Varicella zoster, Epstein-Barr-Virus, Cytomegalovirus, Enterovirus, BK-Virus, JC-Virus, Chlamydia-Phage, Clostridium difficile und Mycoplasma genitalium.

**10.** Verfahren nach Anspruch 8, **dadurch gekennzeichnet, dass** der Mikroorganismus ausgewählt ist aus der Gruppe bestehend aus Chlamydia trachomatis, Clostridium difficile und Mycoplasma genitalium.

**11.** Verwendung eines Kits zum Detektieren einer Ziel-Nukleinsäuresequenz in einer Probe im Verfahren nach einem der Ansprüche 1-10, wobei der Kit mindestens zwei Primer und eine Sonde umfasst, und wobei die mindestens zwei Primer eine Schmelztemperatur (Tm) von mindestens 5°C höher als die Schmelztemperatur (Tm) der Sonde aufweisen.

**12.** Verwendung eines Kits nach Anspruch 11, wobei der Kit weiterhin Desoxyribonukleosidtriphosphate (dNTPs), ein Polymerisations-induzierendes Mittel und eine Gebrauchsanweisung umfasst.

**13.** Verwendung eines Kits nach einem der Ansprüche 11 oder 12, wobei der Kit weiterhin Salze und Puffer umfasst.

**14.** Verwendung eines Kits nach einem der Ansprüche 11 bis 13, wobei der Kit weiterhin eine Ziel-Nukleinsäuresequenz umfasst.

**Revendications**

**1.** Procédé à base de PCR en temps réel destiné à détecter une séquence d'acide nucléique cible dans un échantillon constitué essentiellement de :

a) une étape de préamplification comprenant l'amplification de la séquence d'acide nucléique cible en présence d'au moins deux amorces d'amplification et d'une sonde,
les amorces d'amplification ayant une température de fusion (Tf) d'au moins 5 °C plus élevée que la température de fusion (Tf) de la sonde, et
une telle étape de préamplification étant réalisée à une température plus élevée que la température de fusion (Tf) de la sonde de sorte que les amorces s'hybrident avec la séquence d'acide nucléique cible et la sonde ne s'hybride sensiblement pas,
b) une étape d'amplification et de détection simultanées comprenant une PCR en temps réel qui est réalisée à une température plus basse que la température de l'étape a) permettant une hybridation simultanée des amorces avec une amplification de la séquence d'acide nucléique cible et une hybridation de la sonde avec une détection de la séquence d'acide nucléique cible.

**2.** Procédé selon la revendication 1, dans lequel ladite température de fusion des amorces d'amplification est 10 °C plus élevée que ladite température de fusion de la sonde.

**3.** Procédé selon la revendication 1, dans lequel ladite température de fusion des amorces d'amplification est 20 °C plus élevée que ladite température de fusion de la sonde.

**4.** Procédé selon l'une quelconque des revendications 1 à 3, dans lequel ladite température de fusion des amorces est de 54 à 70 °C et ladite température de fusion de la sonde est de 42 à 48 °C.

**5.** Procédé selon l'une quelconque des revendications 1 à 3, dans lequel ladite température de fusion des amorces est de 64 à 70 °C et ladite température de fusion de la sonde est de 42 à 48 °C.

**6.** Procédé selon l'une quelconque des revendications 1 à 5, **caractérisé en ce que** ladite étape a) de préamplification comprend :

la fourniture d'un mélange de réaction d'amplification comprenant une séquence d'acide nucléique cible, au moins deux amorces, une sonde, un agent induisant une polymérisation, des désoxyribonucléosides triphosphates (dNTP) ;
la soumission dudit mélange de réaction d'amplification à une pluralité de cycles thermiques, comprenant

- une étape de dénaturation permettant de séparer les deux brins de l'acide nucléique cible pour obtenir deux matrices pour la synthèse d'un acide nucléique complémentaire,
- une étape d'hybridation permettant d'anneler chaque amorce avec l'acide nucléique cible correspondant
- une étape d'extension du brin complémentaire à partir de l'amorce par l'agent induisant une polymérisation.

**7.** Procédé selon l'une quelconque des revendications 1 à 6, **caractérisé par le fait que** ladite étape b) de PCR en temps réel de la séquence d'acide nucléique cible comprend

- le traitement du mélange d'amplification contenant la sonde hybridée dans des conditions destinées à amplifier la séquence d'acide nucléique cible,
- la détermination de la quantité dudit signal produit dans l'étape précédente et
- le fait de déterminer si une amplification s'est produite.

**8.** Procédé selon l'une quelconque des revendications 1 à 7, **caractérisé par le fait que** ladite séquence d'acide nucléique cible est une séquence d'ADN d'un micro-organisme, de préférence un micro-organisme pathogène.

**9.** Procédé selon la revendication 8, **caractérisé en ce que** ledit micro-organisme est choisi dans le groupe consistant en *Chlamydia Trachomatis,* le virus de l'hépatite C, le virus de l'hépatite B, *Toxoplasma gondii,* le virus herpès simplex, le virus de la varicelle et du zona, le virus d'Epstein-Barr, le cytomégalovirus, l'entérovirus, le virus BK, le virus JC, un phage de *Chlamydia, Clostridium difficile* et *Mycoplasma genitalium.*

**10.** Procédé selon la revendication 8, **caractérisé en ce que** ledit micro-organisme est choisi dans le groupe consistant en *Chlamydia Trachomatis, Clostridium difficile* et *Mycoplasma genitalium.*

**11.** Utilisation d'un kit destiné à détecter une séquence d'acide nucléique cible dans un échantillon dans le procédé selon l'une quelconque des revendications 1 à 10, dans laquelle ledit kit comprend au moins deux amorces et une sonde, et dans laquelle les au moins deux amorces ont une température de fusion (Tf) d'au moins 5 °C plus élevée que la température de fusion (Tf) de la sonde.

**12.** Utilisation d'un kit selon la revendication 11, ledit kit comprenant en outre des désoxyribonucléosides triphosphates (dNTP), un agent induisant une polymérisation et une notice.

**13.** Utilisation d'un kit selon l'une quelconque des revendications 11 ou 12, ledit kit comprenant en outre des sels et des tampons.

**14.** Utilisation d'un kit selon l'une quelconque des revendications 11 à 13, ledit kit comprenant en outre une séquence d'acide nucléique cible.

FIG. 1

Fig. 2

EP 2 585 615 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006044994 A
- EP 1942196 A
- WO 03054233 A
- WO 2007149903 A
- EP 201184 A
- US 4683202 A
- US 4889818 A
- WO 2010A001132 A

**Non-patent literature cited in the description**

- **SAIKI et al.** *Science,* 1985, vol. 230, 1350
- **SAIKI et al.** *Science,* 1988, vol. 239, 487